# EUROPEAN PATENT APPLICATION

(11) **EP 4 775 585 A1**
(43) Date of publication of application: **15.07.2026**
(21) Application number: 24862728.3
(22) Date of filing: 30.08.2024
(51) Int. Cl.: C07D 487/22, C09K 11/06, H10K 50/11, H10K 50/12, H10K 59/10, H10K 59/60, H10K 59/90, H10K 85/60, H10K 101/30, H10K 101/40

(54) **ORGANIC COMPOUND AND ORGANIC LIGHT-EMITTING ELEMENT**

(30) Priority: 07.09.2023 JP 2023145293
(71) Applicant: Canon Kabushiki Kaisha, Tokyo 146-8501 (JP)
(72) Inventor: SEKIGUCHI Takeshi, Tokyo 146-8501 (JP); FUJISAWA Kaori, Tokyo 146-8501 (JP)
(74) Representative: TBK
(86) International application number: PCT/JP2024/031203
(87) International publication number: WO 2025/053076

(57) **Abstract**

There is provided an organic compound represented by Formula (1): 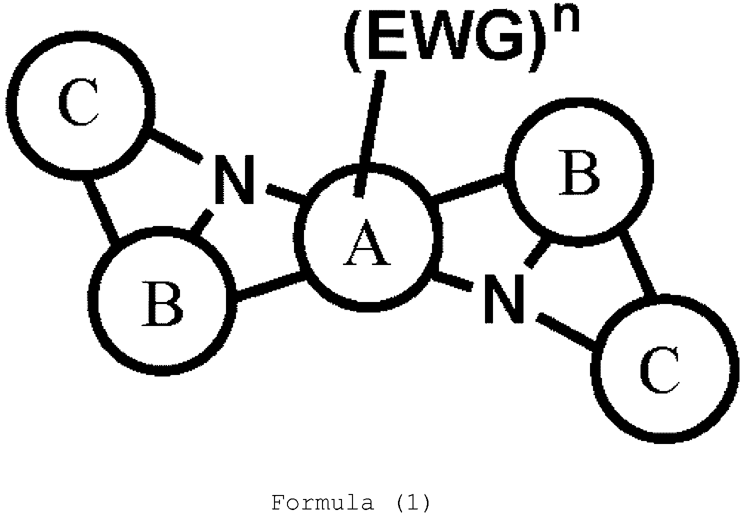 wherein, in Formula (1), Rings A to C are each independently selected from the group consisting of a substituted or unsubstituted aryl group having 6 or more and 50 or less carbon atoms and a substituted or unsubstituted heterocyclic group having 3 or more and 50 or less carbon atoms, EWG is an electron-withdrawing group, an aryl group having at least one electron-withdrawing group, or a heterocyclic group having at least one or more electron-withdrawing groups, and n is an integer of 1 or more.

## Description

### Technical Field

The present invention relates to an organic compound and an organic light-emitting device including the organic compound.

### Background Art

Organic light-emitting devices (hereinafter, also referred to as "organic electroluminescent devices" or "organic EL devices") are electronic devices each including a pair of electrodes and an organic compound layer disposed between these electrodes. The injection of electrons and holes from the pair of electrodes into the organic compound layer generates excitons of the light-emitting organic compound in the organic compound layer, and the organic light-emitting device emits light when the excitons return to a ground state.

To date, the development of compounds suitable for organic light-emitting devices has been actively conducted. Patent Literature 1 discloses Compound Ref-1 as a compound having an indolocarbazole skeleton.

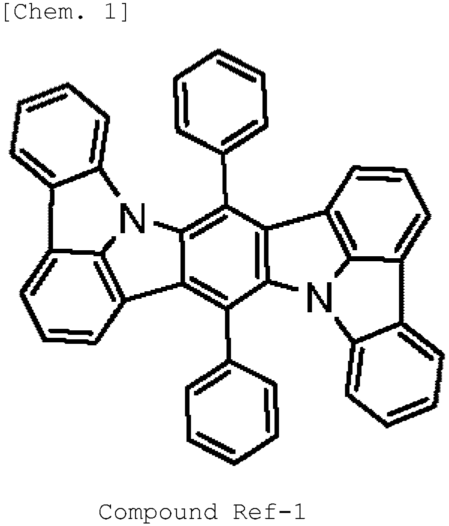

### Citation List

### Patent Literature

PTL 1: International Publication No. WO 2019/111971

### Summary of Invention

### Technical Problem

However, the compounds specifically described in Patent Literature 1 are compounds in which, when the central benzene in each indolocarbazole skeleton has a substituent, the substituent is an aryl group or an aryl group having an alkyl group. Accordingly, there is room for improvement in oxidation stability for the above compounds.

The present invention has been made in view of the above problems, and an object thereof is to provide an organic compound having excellent oxidation stability. Solution to Problem

An organic compound according to the present invention is represented by Formula (1).

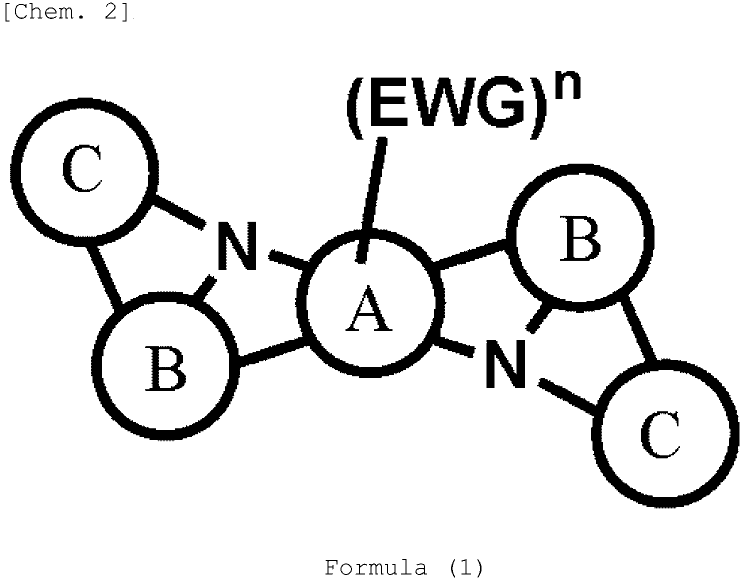

In Formula (1), Rings A to C are each independently selected from the group consisting of a substituted or unsubstituted aryl group having 6 or more and 50 or less carbon atoms and a substituted or unsubstituted heterocyclic group having 3 or more and 50 or less carbon atoms. When any of Rings A to C has a substituent, the substituent is a deuterium atom, a halogen atom, an alkyl group, an aryl group, a heterocyclic group, an alkoxy group, an aryloxy group, a heteroaryloxy group, a silyl group, an amino group, or a cyano group.

EWG is an electron-withdrawing group, an aryl group having at least one electron-withdrawing group, or a heterocyclic group having at least one or more electron-withdrawing groups.

n is an integer of 1 or more.

### Advantageous Effects of Invention

According to the present invention, the organic compound having excellent oxidation stability can be provided.

### Brief Description of Drawings

[Fig. 1A] Fig. 1A is a schematic cross-sectional view illustrating an example of a pixel of a display apparatus according to an embodiment of the present invention.
[Fig. 1B] Fig. 1B is a schematic cross-sectional view of an example of a display apparatus including organic EL devices according to an embodiment of the present invention.
[Fig. 2] Fig. 2 is a schematic view of an example of a display apparatus according to an embodiment of the present invention.
[Fig. 3A] Fig. 3A is a schematic view of an example of an image pickup apparatus according to an embodiment of the present invention.
[Fig. 3B] Fig. 3B is a schematic view of an example of an electronic apparatus according to an embodiment of the present invention.
[Fig. 4A] Fig. 4A is a schematic view of an example of a display apparatus according to an embodiment of the present invention.
[Fig. 4B] Fig. 4B is a schematic view of an example of a foldable display apparatus according to an embodiment of the present invention.
[Fig. 5A] Fig. 5A is a schematic view of an example of a lighting apparatus according to an embodiment of the present invention.
[Fig. 5B] Fig. 5B is a schematic view of an example of an automobile including an automotive lighting unit according to an embodiment of the present invention.
[Fig. 6A] Fig. 6A is a schematic view illustrating an example of a wearable device according to an embodiment of the present invention.
[Fig. 6B] Fig. 6B is a schematic view of an example of a wearable device according to an embodiment of the present invention, the wearable device including an image pickup apparatus.
[Fig. 7A] Fig. 7A is a schematic view of an example of an image-forming apparatus according to an embodiment of the present invention.
[Fig. 7B] Fig. 7B is a schematic view of an example of an exposure light source of an image-forming apparatus according to an embodiment of the present invention.
[Fig. 7C] Fig. 7C is a schematic view of an example of an exposure light source of an image-forming apparatus according to an embodiment of the present invention.
[Fig. 8] Fig. 8 is a view illustrating the HOMO orbital distribution and the LUMO orbital distribution of Compound A-1, Compound A-2, Compound Ref-4, and Compound Ref-5.

### Description of Embodiments

In the present specification, examples of a halogen atom include, but are not limited to, a fluorine atom, a chlorine atom, a bromine atom, and an iodine atom.

An alkyl group may have 1 or more and 20 or less carbon atoms, 1 or more and 10 or less carbon atoms, or 1 or more and 4 or less carbon atoms. Specific examples thereof include, but are not limited to, a methyl group, an ethyl group, a n-propyl group, an isopropyl group, a n-butyl group, a tert-butyl group, a sec-butyl group, an octyl group, a cyclohexyl group, a tert-pentyl group, a 3-methylpentan-3-yl group, a 1-adamantyl group, and a 2-adamantyl group.

An aryl group may have 6 or more and 50 or less carbon atoms, 6 or more and 20 or less carbon atoms, 6 or more and 14 or less carbon atoms, or 6 or more and 12 or less carbon atoms. Specific examples thereof include, but are not limited to, a phenyl group, a naphthyl group, an indenyl group, a biphenyl group, a terphenyl group, a fluorenyl group, a phenanthryl group, a triphenylenyl group, a pyrenyl group, an anthranyl group, a perylenyl group, a chrysenyl group, and a fluoranthenyl group.

A heterocyclic group may have 3 or more and 50 or less carbon atoms, 3 or more and 20 or less carbon atoms, 3 or more and 17 or less carbon atoms, or 3 or more and 12 or less carbon atoms. Specific examples thereof include, but are not limited to, a pyridyl group, a pyrimidyl group, a pyrazyl group, a triazyl group, a benzofuranyl group, a benzothiophenyl group, a dibenzofuranyl group, a dibenzothiophenyl group, an oxazolyl group, an oxadiazolyl group, a thiazolyl group, a thiadiazolyl group, a carbazolyl group, an acridinyl group, and a phenanthrolyl group.

An amino group may be a substituted amino group substituted with an alkyl group or an aryl group, and may be a substituted amino group substituted with an alkyl group having 1 or more and 4 or less carbon atoms or an aryl group having 6 or more and 12 or less carbon atoms. Specific examples thereof include, but are not limited to, an N-methylamino group, an N-ethylamino group, an N,N-dimethylamino group, an N,N-diethylamino group, an N-methyl-N-ethylamino group, an N-benzylamino group, an N-methyl-N-benzylamino group, an N,N-dibenzylamino group, an anilino group, an N,N-diphenylamino group, an N,N-dinaphthylamino group, an N,N-difluorenylamino group, an N-phenyl-N-tolylamino group, an N,N-ditolylamino group, an N-methyl-N-phenylamino group, an N,N-dianisolylamino group, an N-mesityl-N-phenylamino group, an N,N-dimesitylamino group, an N-phenyl-N-(4-tert-butylphenyl)amino group, an N-phenyl-N-(4-trifluoromethylphenyl)amino group, and an N-piperidyl group.

An alkoxy group may have 1 or more and 10 or less carbon atoms, or 1 or more and 4 or less carbon atoms. Specific examples thereof include, but are not limited to, a methoxy group, an ethoxy group, a propoxy group, a 2-ethyl-octyloxy group, and a benzyloxy group.

A specific example of an aryloxy group is a phenoxy group, but is not limited thereto.

A specific example of a heteroaryloxy group is a thienyloxy group, but is not limited thereto.

Specific examples of a silyl group include, but are not limited to, a trimethylsilyl group and a triphenylsilyl group.

Examples of substituents that may further be contained in the alkyl group, the alkoxy group, the amino group, the aryloxy group, the silyl group, the aryl group, and the heterocyclic group include, but are not limited to, halogen atoms, such as fluorine, chlorine, bromine, and iodine; alkyl groups, such as a methyl group, an ethyl group, a n-propyl group, an isopropyl group, a n-butyl group, and a tert-butyl group; alkoxy groups, such as a methoxy group, an ethoxy group, a propoxy group; amino groups, such as a dimethylamino group, a diethylamino group, a dibenzylamino group, a diphenylamino group, and a ditolylamino group; aryloxy groups, such as a phenoxy group; aryl groups, such as a phenyl group and a biphenyl group; heterocyclic groups, such as a pyridyl group and pyrrolyl group; and a cyano group.

### (1) Organic Compound

First, an organic compound according to the present invention will be described.

The organic compound according to the present invention is an organic compound represented by Formula (1).

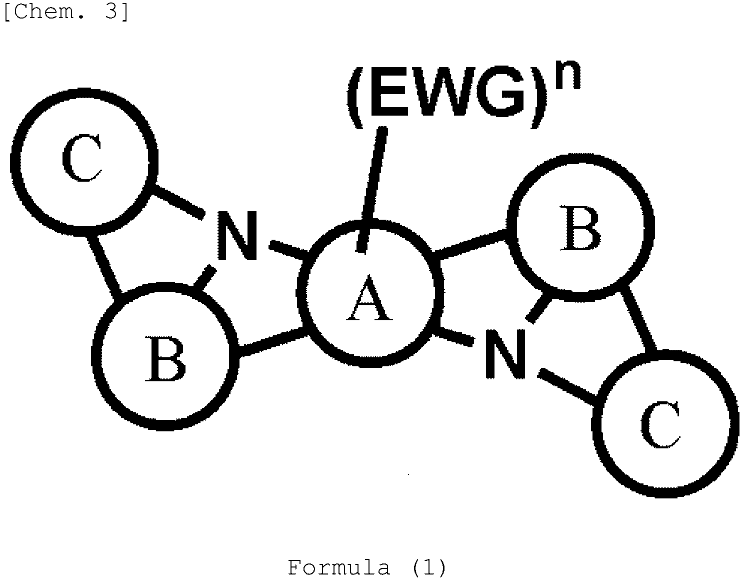

In Formula (1), Rings A to C are each independently selected from the group consisting of a substituted or unsubstituted aryl group having 6 or more and 50 or less carbon atoms and a substituted or unsubstituted heterocyclic group having 3 or more and 50 or less carbon atoms.

When any of Rings A to C has a substituent, the substituent is a deuterium atom, a halogen atom, an alkyl group, an aryl group, a heterocyclic group, an alkoxy group, an aryloxy group, a heteroaryloxy group, a silyl group, an amino group, or a cyano group.

When any of Rings A to C has a substituent, the substituent may be an alkyl group, an aryl group, an amino group, or a heterocyclic group, and is preferably an alkyl group, an aryl group, or a heterocyclic group.
Specifically, the substituent may be an alkyl group having 1 or more and 4 or less carbon atoms, an aryl group having 6 or more and 14 or less carbon atoms, or a heterocyclic group having 3 or more and 12 or less carbon atoms. More specifically, the substituent may be a methyl group, an iso-propyl group, a tert-butyl group, a phenyl group, a phenyl group having a tert-butyl group, a phenyl group having a methyl group, or a dibenzofuranyl group. The amino group may be a diphenylamino group, and any of the phenyl groups in the diphenylamino group may have an alkyl group having 1 or more and 4 or less carbon atoms. The alkyl group may specifically be an iso-propyl group or a tert-butyl group.

Rings A to C may each be a substituted or unsubstituted aryl group having 6 or more and 20 or less carbon atoms or a substituted or unsubstituted heterocyclic group having 3 or more and 17 or less carbon atoms.

Specifically, Ring A may be a benzene skeleton, a naphthalene skeleton, or a fluorene skeleton, and may be a benzene skeleton or a naphthalene skeleton. Each Ring B may be a benzene skeleton, a benzofuran skeleton, or a benzothiophene skeleton, and may be a benzene skeleton or a benzofuran skeleton. Each Ring C may be a benzene skeleton, a benzofuran skeleton, or a benzothiophene skeleton, and may be a benzene skeleton or a benzofuran skeleton.

EWG is an electron-withdrawing group, an aryl group having at least one electron-withdrawing group, or a heterocyclic group having at least one or more electron-withdrawing groups. EWG may be an aryl group having at least one electron-withdrawing group and having 6 or more and 20 or less carbon atoms, or a heterocyclic group having at least one or more electron-withdrawing groups and having 3 or more and 17 or less carbon atoms.

Here, the electron-withdrawing group is a trifluoromethyl group, a trichloromethyl group, a nitro group, a cyano group, an aldehyde group, a ketone group, an ester group, a carboxylic acid group, a sulfonyl group, a sulfonic acid group, a pyridyl group, a triazinyl group, a halogen atom, or the like. Among these, the Hammett substituent constant of the electron-withdrawing group is preferably 0.01 or more and 0.70 or less. More specifically, the electron-withdrawing group is preferably a trifluoromethyl group, a cyano group, or a fluorine atom.

A bipyridyl group is defined as a structure in which a pyridyl group, which is a heterocyclic group, has another pyridyl group that functions as an electron-withdrawing group. Similarly, a benzonitrile group is defined as a structure in which a phenyl group, which is an aryl group, has a cyano group that functions as an electron-withdrawing group.

n is an integer of 1 or more. The possible values of n are defined by the structure of Ring A. For example, when Ring A is a benzene skeleton, n is 1 or 2. When Ring B is a naphthalene skeleton, n is an integer of 1 or more and 4 or less.

Formula (1) may be a structure represented by Formula (2) or (3) below. Formula (2) is a compound in which Ring A in Formula (1) is a benzene skeleton. Formula (3) is a compound in which Ring A in Formula (1) is a naphthalene skeleton.

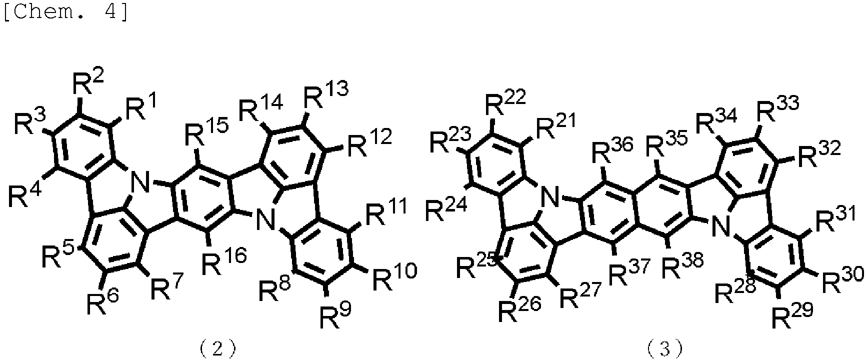

In Formula (2), R¹ to R¹⁶ are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted alkoxy group, a substituted or unsubstituted aryloxy group, a substituted or unsubstituted heteroaryloxy group, a substituted or unsubstituted silyl group, a substituted or unsubstituted amino group, and a cyano group. R¹ and R², R² and R³, R³ and R⁴, R⁵ and R⁶, R⁶ and R⁷, R⁸ and R⁹, R⁹ and R¹⁰, R¹⁰ and R¹¹, R¹² and R¹³, or R¹³ and R¹⁴ may be taken together to form a ring. Among these, it is preferred that R³ and R⁴ be taken together to form a ring and that R¹⁰ and R¹¹ be taken together to form a ring. It is preferred that R⁵ and R⁶ be taken together to form a ring and that R¹² and R¹³ be taken together to form a ring. Each ring thus formed may be a substituted or unsubstituted aryl group having 6 or more and 12 or less carbon atoms or a substituted or unsubstituted heterocyclic group having 3 or more and 12 or less carbon atoms. Specifically, each ring thus formed may be a substituted or unsubstituted benzofuran skeleton or a substituted or unsubstituted benzothiophene skeleton. Provided that at least one of R¹⁵ and R¹⁶ is an electron-withdrawing group, an aryl group having at least one or more electron-withdrawing groups, or a heterocyclic group having at least one or more electron-withdrawing groups.

In Formula (2), when the organic compound according to the present invention has a substituent, any of R³, R⁴, R⁵, R⁶, R¹⁰, R¹¹, R¹², and R¹³ may have a substituent. R³, R⁶, R¹⁰, and R¹³, or any of the pairs of R³ and R¹⁰, R⁴ and R¹¹, and R⁵ and R¹² may have a substituent.

In Formula (3), R²¹ to R³⁸ are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted alkoxy group, a substituted or unsubstituted aryloxy group, a substituted or unsubstituted heteroaryloxy group, a substituted or unsubstituted silyl group, a substituted or unsubstituted amino group, and a cyano group. R²¹ and R²², R²² and R²³, R²³ and R²⁴, R²⁴ and R²⁵, R²⁶ and R²⁷, R²⁸ and R²⁹, R²⁹ and R³⁰, R³⁰ and R³¹, R³² and R³³, or R³³ and R³⁴ may be taken together to form a ring. Among these, it is preferred that R²³ and R²⁴ be taken together to form a ring and that R³⁰ and R³¹ be taken together to form a ring. It is preferred that R²⁵ and R²⁶ be taken together to form a ring and that R³² and R³³ be taken together to form a ring. Each ring thus formed may be a substituted or unsubstituted aryl group having 6 or more and 12 or less carbon atoms or a substituted or unsubstituted heterocyclic group having 3 or more and 12 or less carbon atoms. Specifically, each ring thus formed may be a substituted or unsubstituted benzofuran skeleton or a substituted or unsubstituted benzothiophene skeleton. Provided that at least one of R³⁵ to R³⁸ is an electron-withdrawing group, an aryl group having at least one or more electron-withdrawing groups, or a heterocyclic group having at least one or more electron-withdrawing groups.

In Formula (3), when the organic compound according to the present invention has a substituent, any of R²³, R²⁴, R²⁵, R²⁶, R³⁰, R³¹, R³², and R³³ may have a substituent. R²³, R²⁶, R³⁰, and R³³, or any of the pairs of R²³ and R³⁰, R²⁴ and R³¹, and R²⁵ and R³², may have a substituent.

The characteristics of the organic compound according to the present invention will be described below.

In the organic compound according to the present invention, Ring A in Formula (1) has an electron-withdrawing group, an aryl group having an electron-withdrawing group, or a heterocyclic group having an electron-withdrawing group, so that the highest occupied molecular orbital (HOMO) is low (further from the vacuum level). As a result, the organic compound according to the present invention has excellent oxidation stability.

Here, the HOMO values of Compound A-1 and Compound A-2, which are examples of the organic compound according to the present invention, and Compound Ref-1, which is a comparative compound, were calculated using Gaussian 16 (Gaussian 16, Revision C.01, M. J. Frisch et al., Gaussian, Inc., Wallingford, CT, 2019), molecular orbital calculation software developed by Gaussian, Inc., USA. The keyword used was B3LYP/6-31G*. The results are presented in Table 1.

### [Table 1]

**Table 1**

| Compound | Compound A-1 | Compound A-2 | Compound Ref-1 |
|---|---|---|---|
| Structural formula | 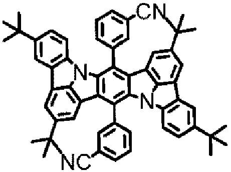 | 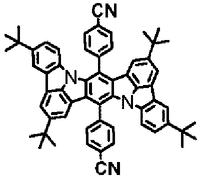 | 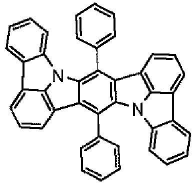 |
| HOMO/eV | -5.25 | -5.29 | -5.11 |

As can be seen from Table 1, the HOMOs of Compounds A-1 and A-2, which are examples of the organic compounds according to the present invention, are -5.25 eV and -5.29 eV, respectively, whereas the HOMO of compound Ref-1, which is a comparative compound, is -5.11 eV. This is considered to be because the organic compound according to the present invention has an electron-withdrawing group. Therefore, compared with the HOMO of Compound Ref-1, the organic compound according to the present invention is an organic compound having excellent oxidation stability.

The organic compound according to the present invention has at least one of an electron-withdrawing group, an aryl group having an electron-withdrawing group, and a heterocyclic group having an electron-withdrawing group, and thus can achieve the effects of the present invention. The HOMO values of Compounds A-2, A-3, A-4, and A-5, which are examples of the organic compounds according to the present invention, and Compounds Ref-2 and Ref-3, which are comparative compounds, were determined using the calculation method described above. The results are presented in Table 2.

### [Table 2]

**Table 2**

| Compound | Compound A-2 | Compound A-3 | Compound A-4 |
|---|---|---|---|
| Structural formula | 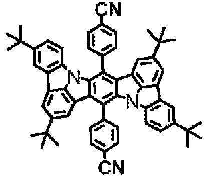 | 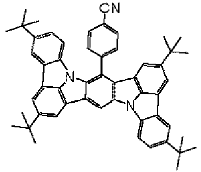 | 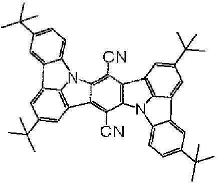 |
| HOMO/eV | -5.29 | -5.18 | -5.53 |

| Compound | Compound A-5 | Compound Ref-2 | Compound Ref-3 |
|---|---|---|---|
| Structural formula | 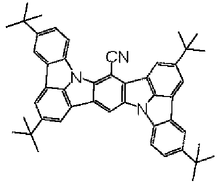 | 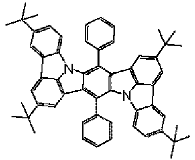 | 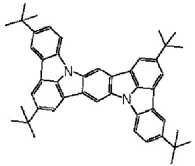 |
| HOMO/eV | -5.30 | -4.94 | -5.03 |

As can be seen from Table 2, each organic compound according to the present invention exhibited a lower HOMO value than those of Comparative Compounds Ref-2 and Ref-3. Each organic compound according to the present invention has at least one of an electron-withdrawing group, an aryl group having an electron-withdrawing group, or a heterocyclic group having an electron-withdrawing group, and thus can achieve the effects of the present invention.

In the organic compound according to the present invention, Ring A in Formula (1) has an electron-withdrawing group, an aryl group having an electron-withdrawing group, or a heterocyclic group having an electron-withdrawing group; thus, the full width at half maximum of the emission spectrum should exhibit a smaller value.

Patent Literature 1 describes Compounds Ref-4 and Ref-5 below. These are compounds in which each Ring B in Formula (1) has an aryl group having an electron-withdrawing group.

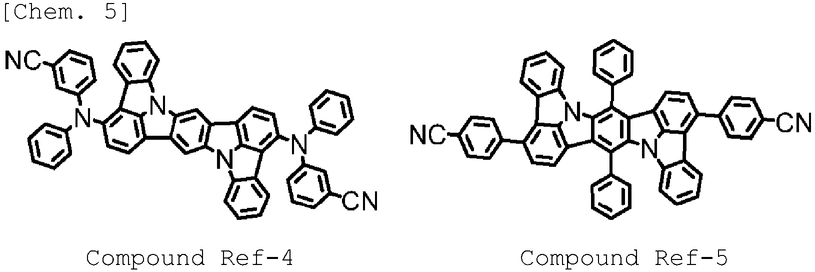

The organic compound according to the present invention differs from Compounds Ref-4 and Ref-5 in that Ring A in Formula (1) has an electron-withdrawing group, an aryl group having an electron-withdrawing group, or a heterocyclic group having an electron-withdrawing group. Accordingly, the organic compound according to the present invention can be expected to exhibit superior color purity compared with Compounds Ref-4 and Ref-5.

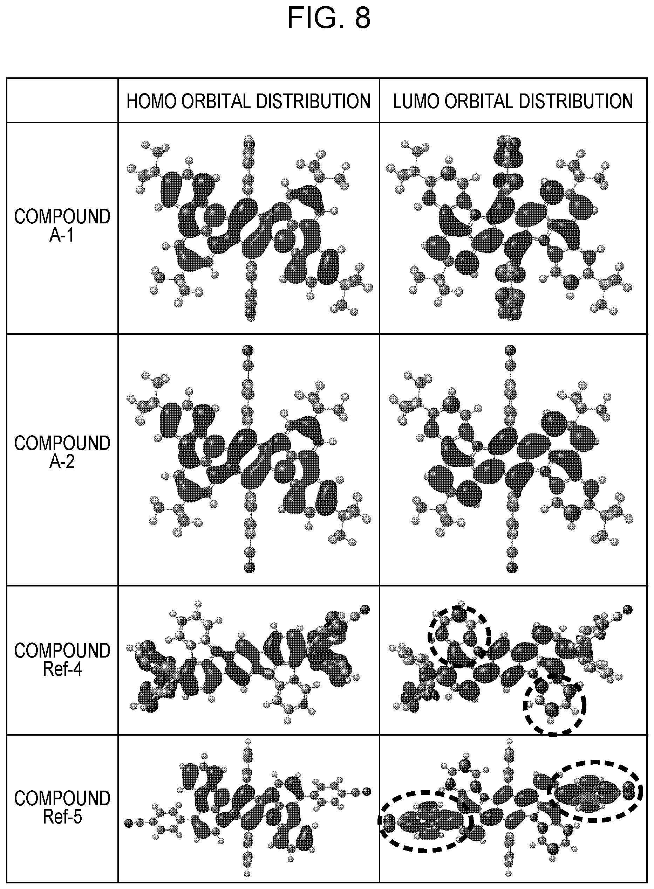

The HOMO orbital distribution and the LUMO orbital distribution of each of Compounds A-1 and A-2, which are examples of the organic compound according to the present invention, and of each of Compounds Ref-4 and Ref-5, which are comparative compounds, were visualized using Gaussian 16 (Gaussian 16, Revision C.01, M. J. Frisch et al., Gaussian, Inc., Wallingford, CT, 2019), the molecular orbital calculation software manufactured by Gaussian, Inc., USA. In this calculation, the isosurface value (electron density isosurface) was set to 0.02. The results are presented in Fig. 8.

As illustrated in Fig. 8, the HOMO orbital distribution and the LUMO orbital distribution of each of Compounds A-1 and A-2, which are examples of the organic compound according to the present invention, are localized in the middle of the structure represented by Formula (1). In contrast, a difference is observed between the HOMO orbital distribution and the LUMO orbital distribution of each of Comparative Compounds Ref-4 and Ref-5 (circled by the black dotted lines in Fig. 8). In other words, the overlap between the HOMO orbital distribution and the LUMO orbital distribution of each of Comparative Compounds Ref-4 and Ref-5 is small, compared with that of each organic compound according to the present invention.

Typically, when the overlap between the HOMO orbital distribution and the LUMO orbital distribution is small, the structural relaxation between the excited state and the ground state increases, and thus the emission spectrum tends to broaden. On the other hand, when the overlap between the HOMO orbital distribution and the LUMO orbital distribution is large, the structural relaxation between the excited state and the ground state can be inhibited, and thus the emission spectrum tends to be sharp.

Therefore, each organic compound according to the present invention is expected to exhibit a sharp emission spectrum, compared with Comparative Compounds Ref-4 and Ref-5. In other words, each organic compound according to the present invention is expected to exhibit superior color purity.

More preferred embodiments of the organic compound according to the present invention will be described below.

Among the organic compounds according to the present invention, in Formula (1), EWG is preferably an aryl group having at least one electron-withdrawing group. The oscillator strengths of Compounds A-1, A-4, and A-6, which are examples of the organic compounds according to the present embodiment, were calculated using Gaussian 16 (Gaussian 16, Revision C.01, M. J. Frisch et al., Gaussian, Inc., Wallingford, CT, 2019), molecular orbital calculation software developed by Gaussian, Inc., USA. The keyword used was B3LYP/6-31G*. The results are presented in Table 3.

### [Table 3]

**Table 3**

| Compound | Compound A-1 | Compound A-4 | Compound A-6 |
|---|---|---|---|
| Structural formula | 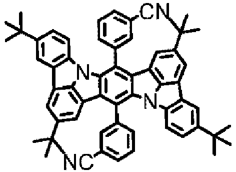 | 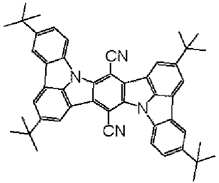 | 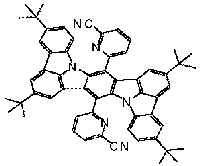 |
| Oscillator strength | 0.16 | 0.02 | 0.01 |

From Table 3, it can be seen that the oscillator strength of Compound A-1 is higher than those of Compounds A-4 and A-6.

Here, the relationship between oscillator strength and quantum yield (luminous efficiency) is described. As described in paragraph [0262] of Japanese Patent Laid-Open No. 2020-47930 and paragraph [0035] of Japanese Patent Laid-Open No. 2022-46999, it is known that compounds with high oscillator strengths exhibit high quantum yields (luminous efficiencies).

Therefore, among the organic compounds according to the present invention, EWG in Formula (1) is preferably an aryl group having at least one electron-withdrawing group from the viewpoint of the quantum yield (luminous efficiency).

Among the organic compounds according to the present invention, when EWG in Formula (1) is an aryl group having at least one electron-withdrawing group, the aryl group is preferably a benzene skeleton. This is because the oscillator strength exhibits a high value, compared with the case where the aryl group having at least one electron-withdrawing group is a naphthalene skeleton. The oscillator strengths of Compounds A-1 and A-7, which are examples of the organic compound according to the present embodiment, were determined using the same calculation method as in Table 3. The results are presented in Table 4.

### [Table 4]

**Table 4**

| Compound | Compound A-1 | Compound A-7 |
|---|---|---|
| Structural formula | 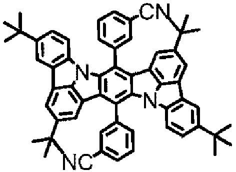 | 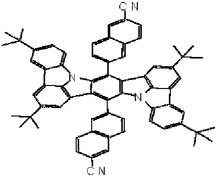 |
| Oscillator strength | 0.16 | 0.02 |

From Table 4, it was found that Compound A-1 exhibited a higher oscillator strength than Compound A-7. As described above, it is known that a high oscillator strength leads to a high quantum yield (luminous efficiency). Therefore, from the viewpoint of the quantum yield (luminous efficiency), the aryl group having at least one electron-withdrawing group is preferably a benzene skeleton.

Among the organic compounds according to the present invention, when EWG in Formula (1) is an aryl group having at least one electron-withdrawing group and the aryl group is a benzene skeleton, the electron-withdrawing group is preferably bonded to the meta-position or para-position relative to Ring A. This is because, in this case, the organic compound according to the present embodiment exhibits a higher oscillator strength. The oscillator strengths of Compounds A-1, A-2, and A-8, which are examples of the organic compound according to the present embodiment, were determined using the same calculation method as in Table 3. The results are presented in Table 5.

### [Table 5]

**Table 5**

| Compound | Compound A-1 | Compound A-2 | Compound A-8 |
|---|---|---|---|
| Structural formula | 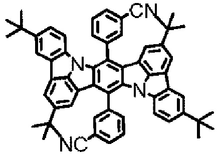 | 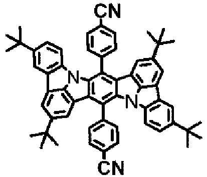 | 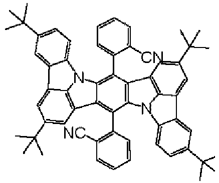 |
| Oscillator strength | 0.16 | 0.16 | 0.12 |

Table 5 indicated that Compounds A-1 and A-2 exhibit higher oscillator strengths than Compound A-8. As described above, it is known that a high oscillator strength leads to a high quantum yield (luminous efficiency). Therefore, from the viewpoint of the quantum yield (luminous efficiency), when EWG in Formula (1) is an aryl group having at least one electron-withdrawing group and the aryl group is a benzene skeleton, the electron-withdrawing group is preferably bonded to the meta-position or para-position relative to Ring A.

Among the organic compounds according to the present invention, the electron-withdrawing group is preferably a trifluoromethyl group, a cyano group, or a fluorine atom. This is because these electron-withdrawing groups have a moderately strong electron-withdrawing property, and therefore the overlap between the HOMO orbital distribution and the LUMO orbital distribution is larger. As a result, the organic compound according to the present embodiment is expected to be an organic compound that exhibits a smaller full width at half maximum. In other words, the organic compound according to the present embodiment is expected to be an organic compound with superior color purity.

Specific examples of the organic compound according to the present invention are illustrated below. However, the present invention is not limited thereto.

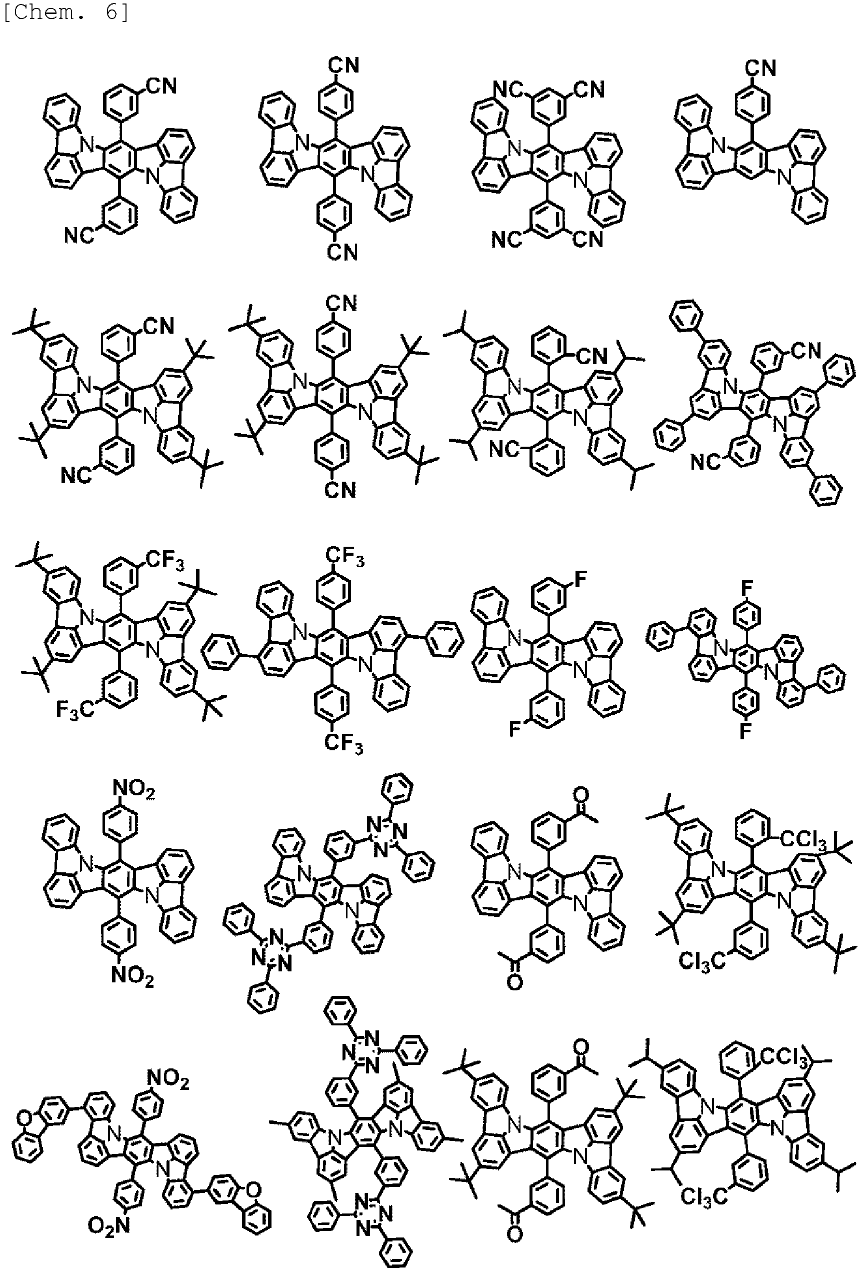

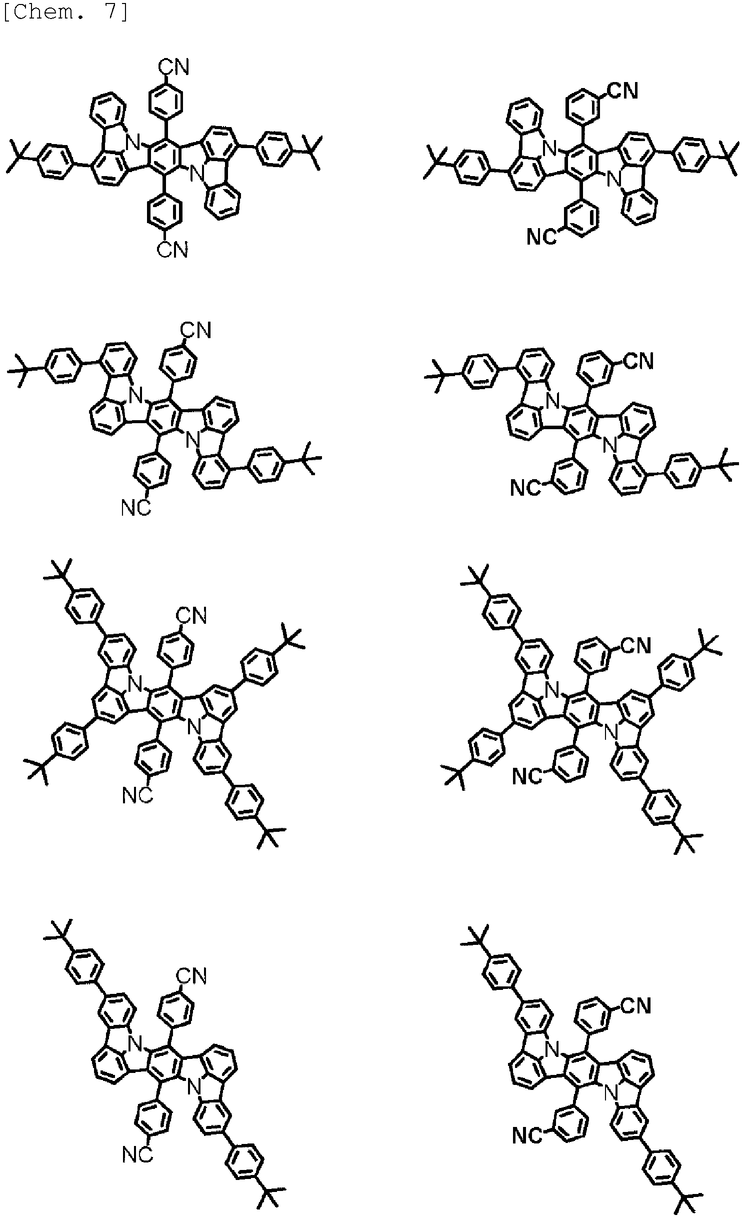

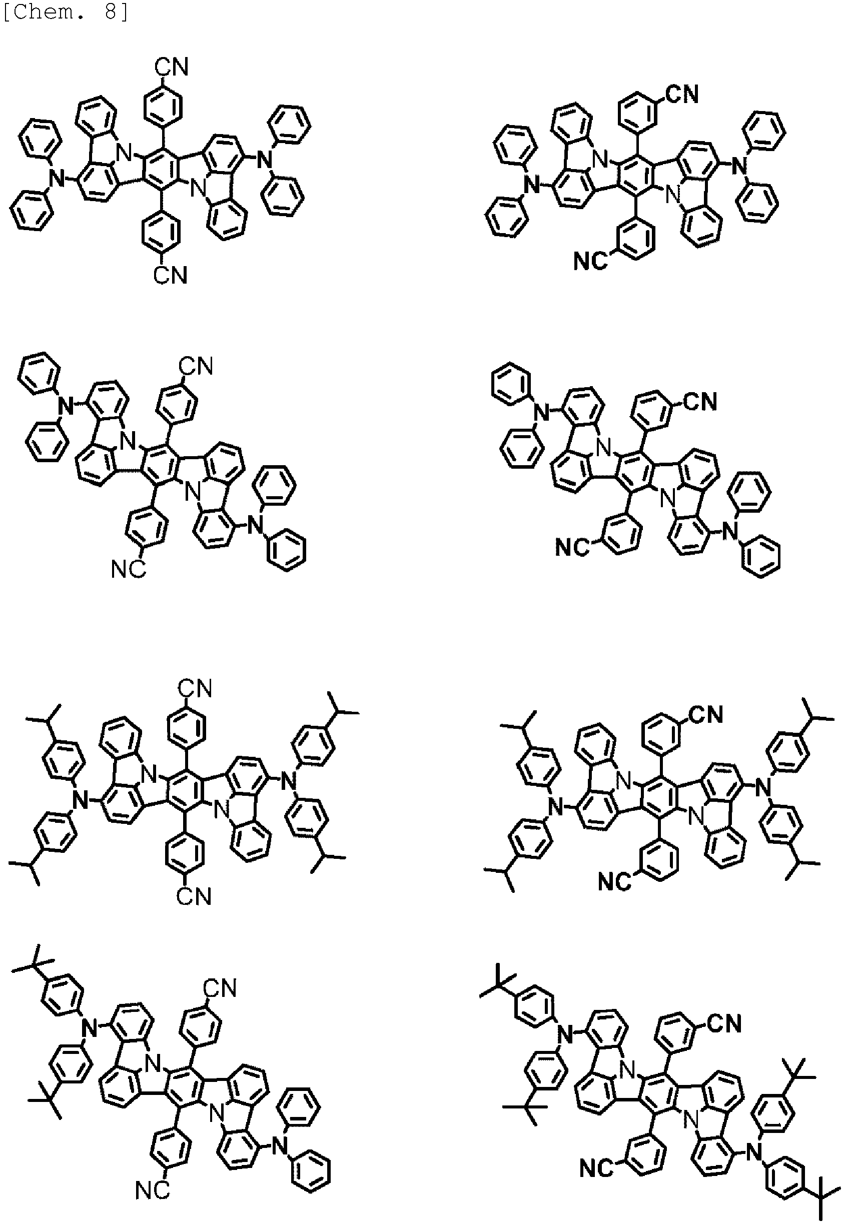

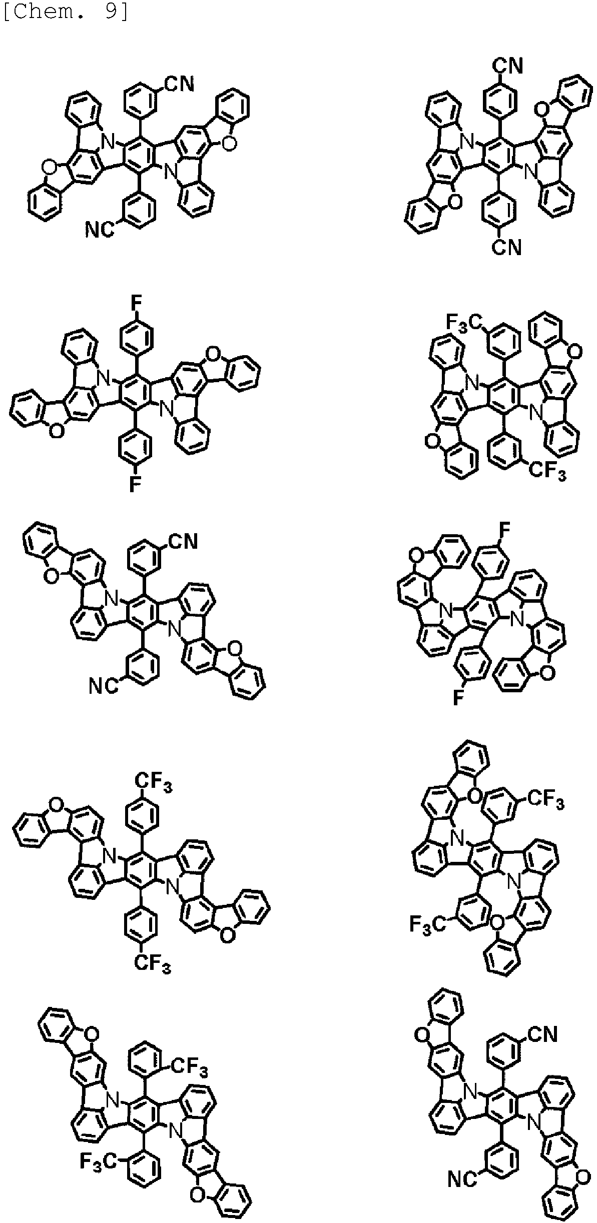

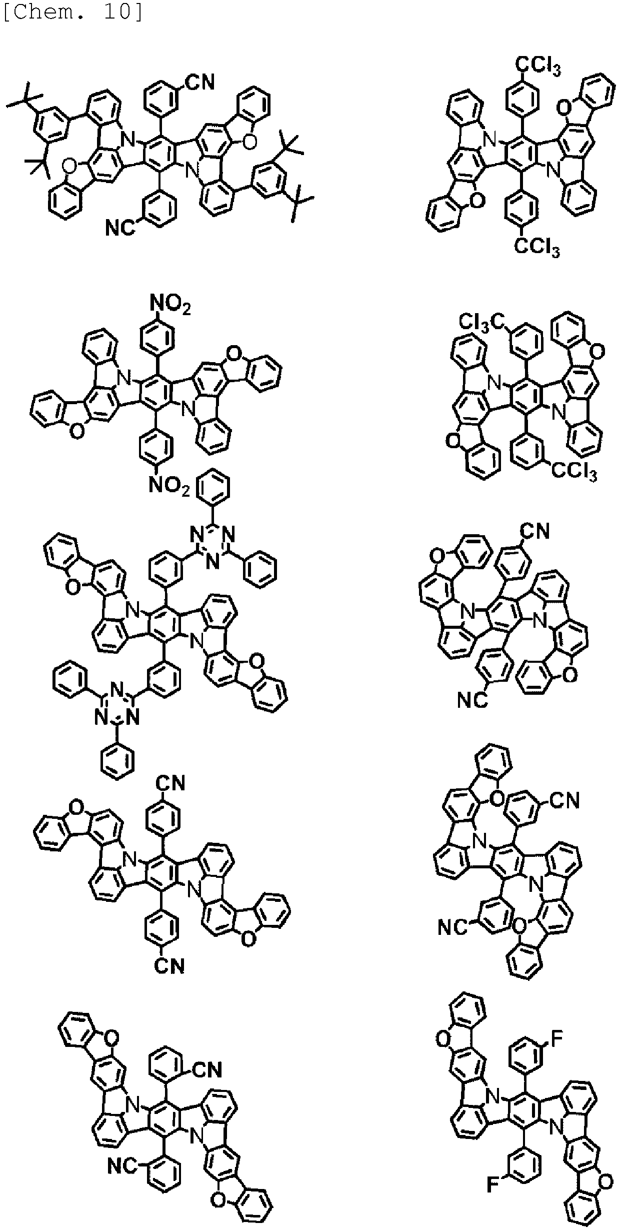

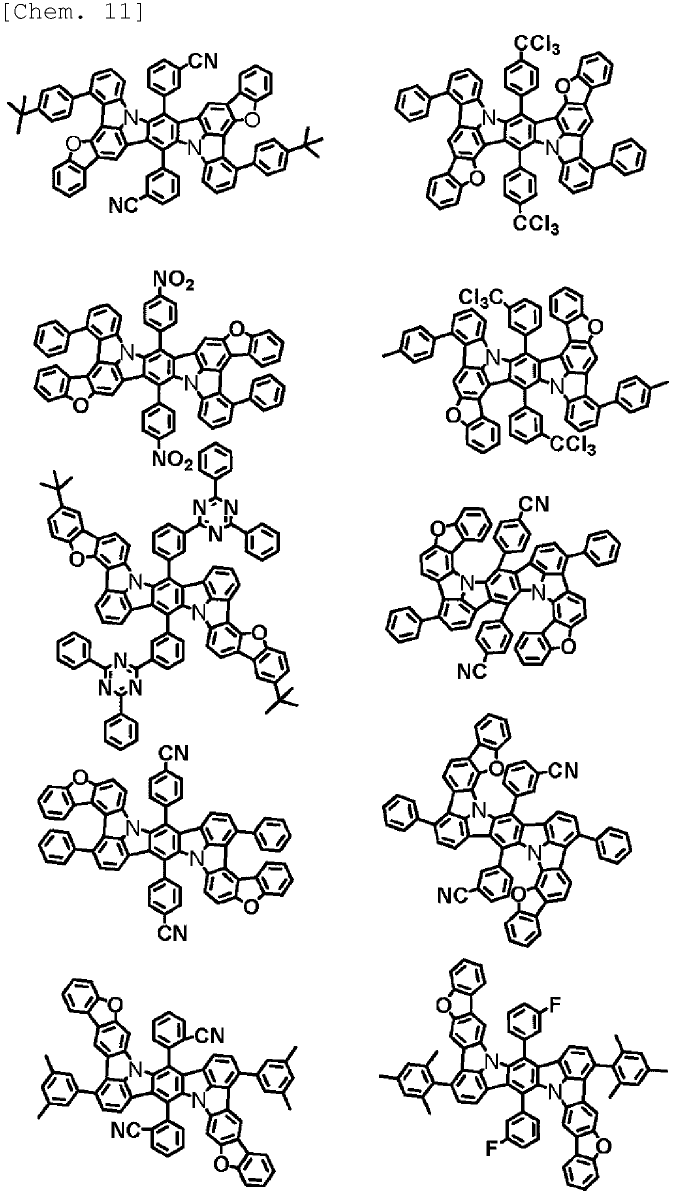

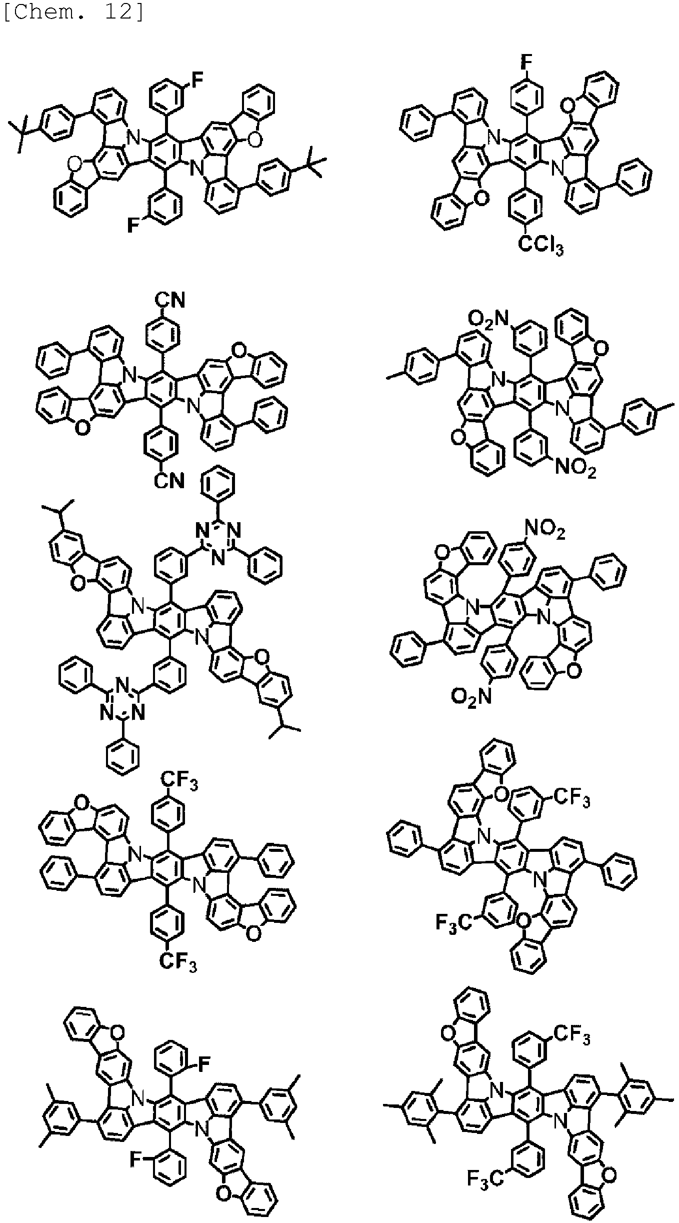

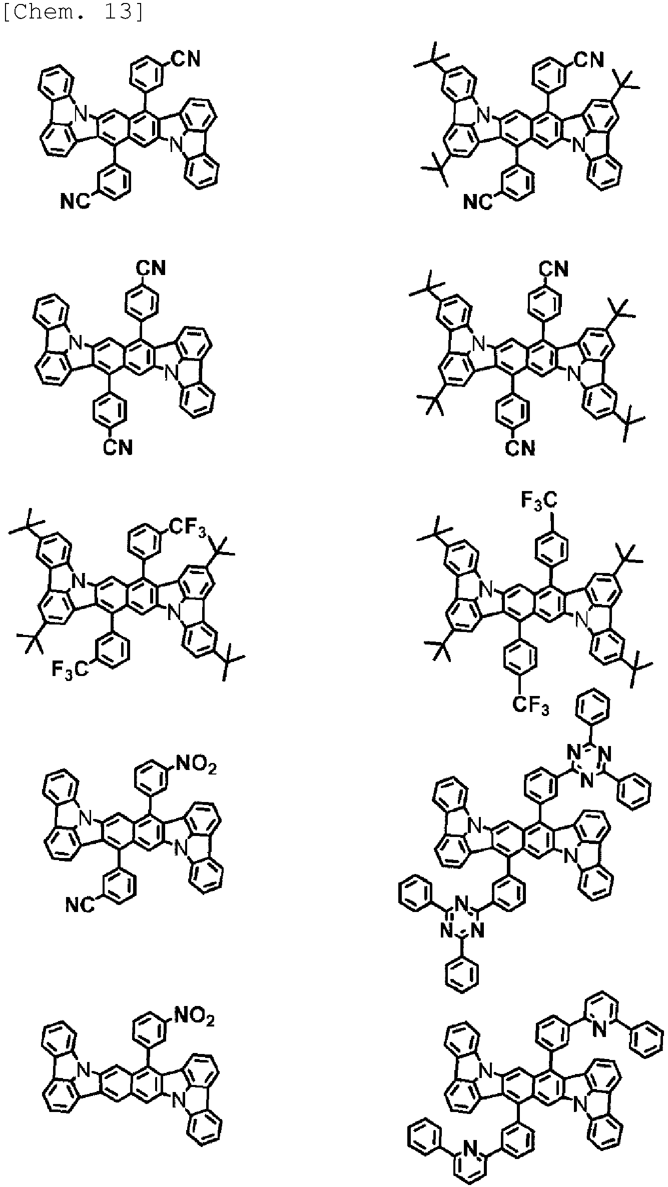

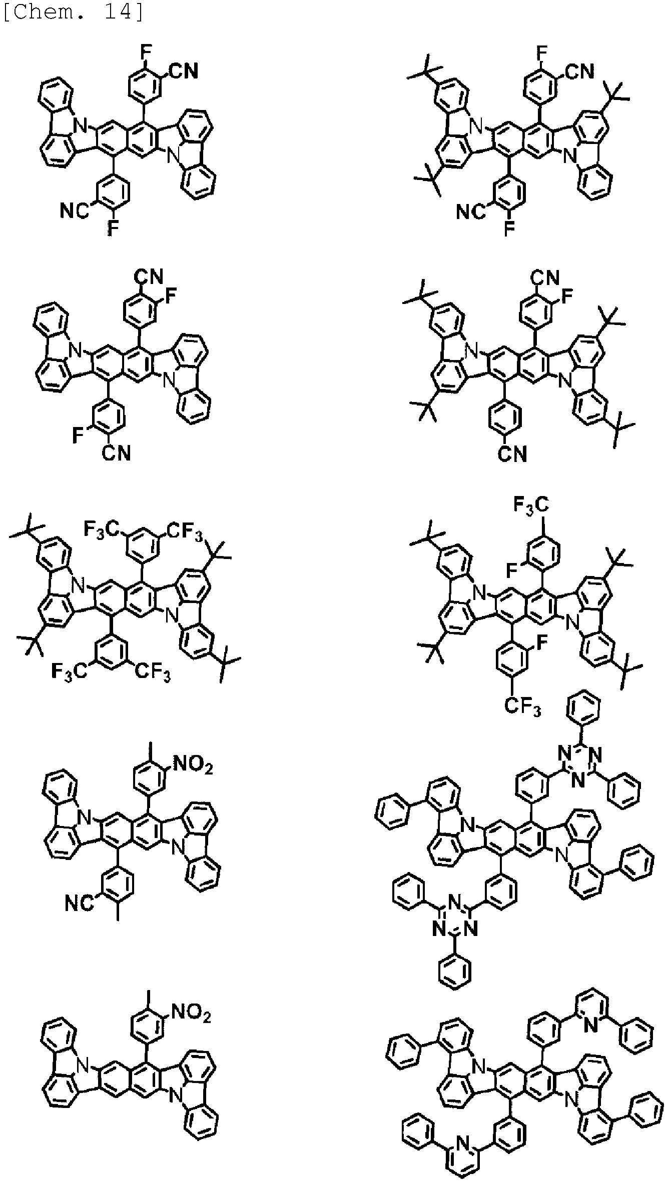

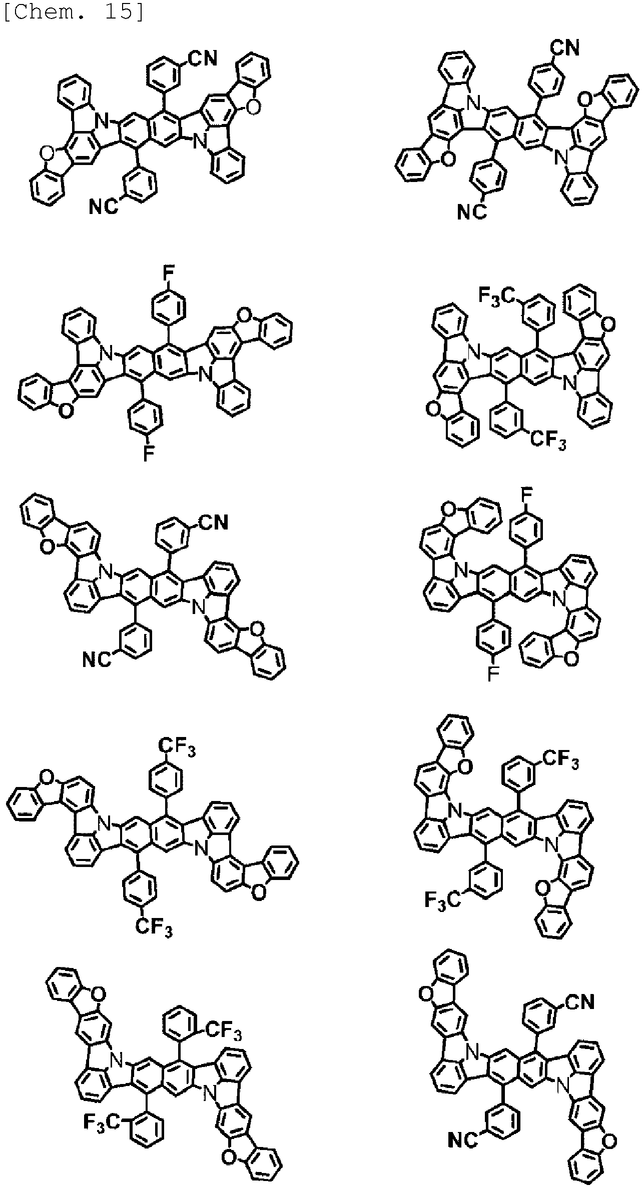

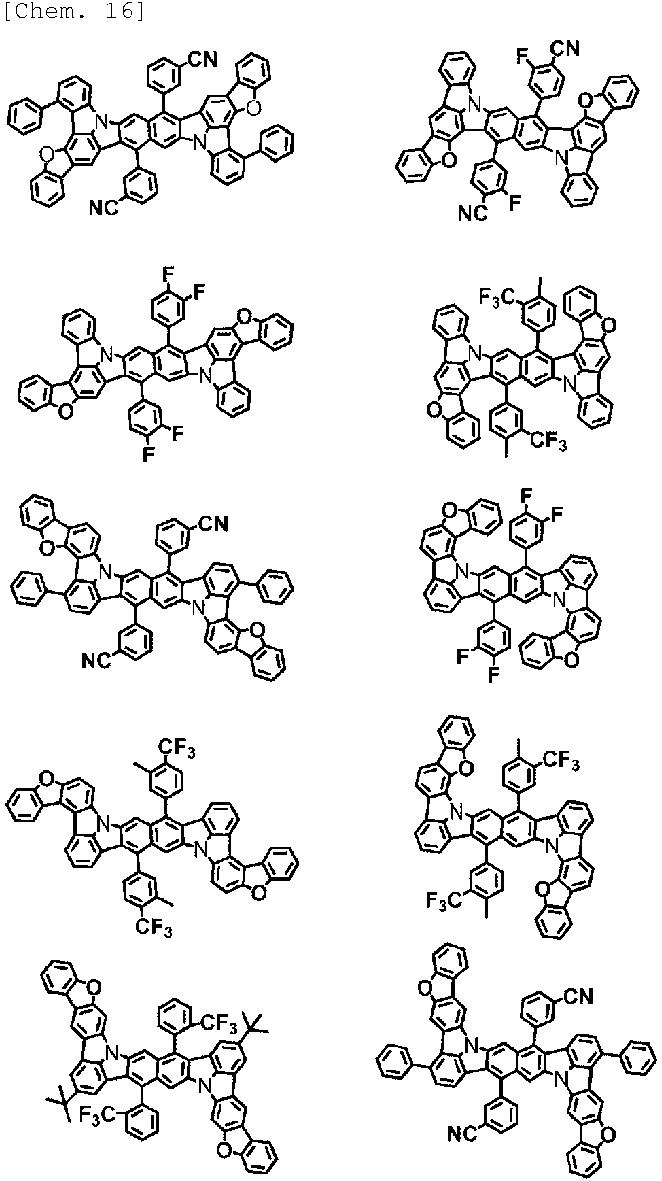

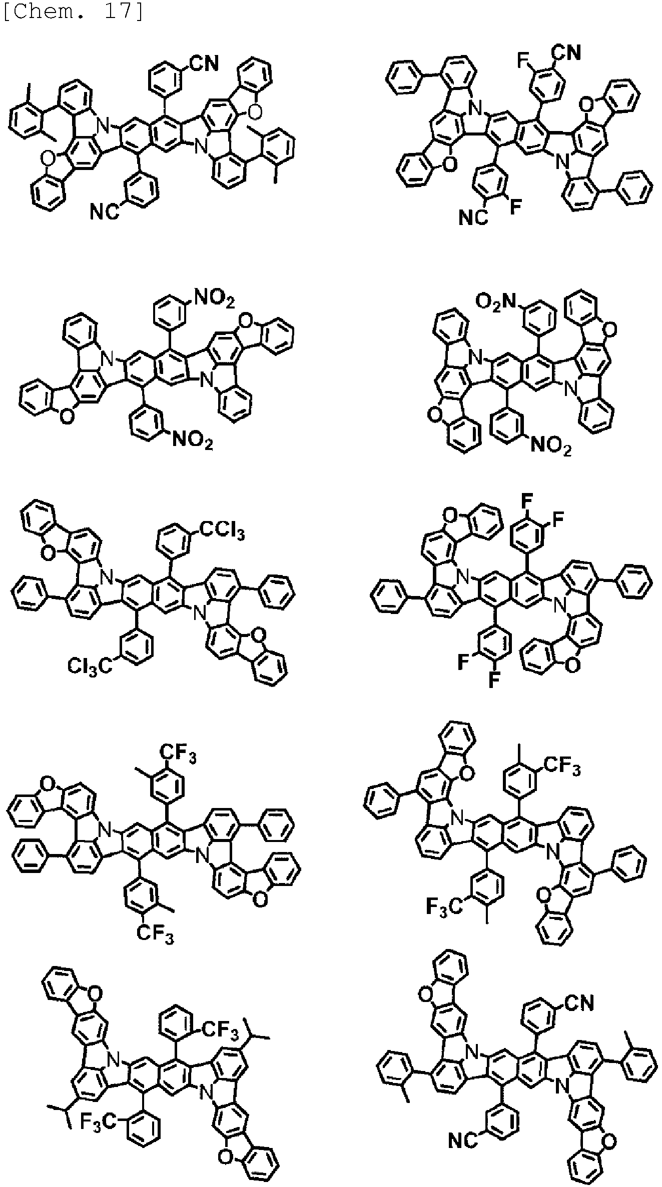

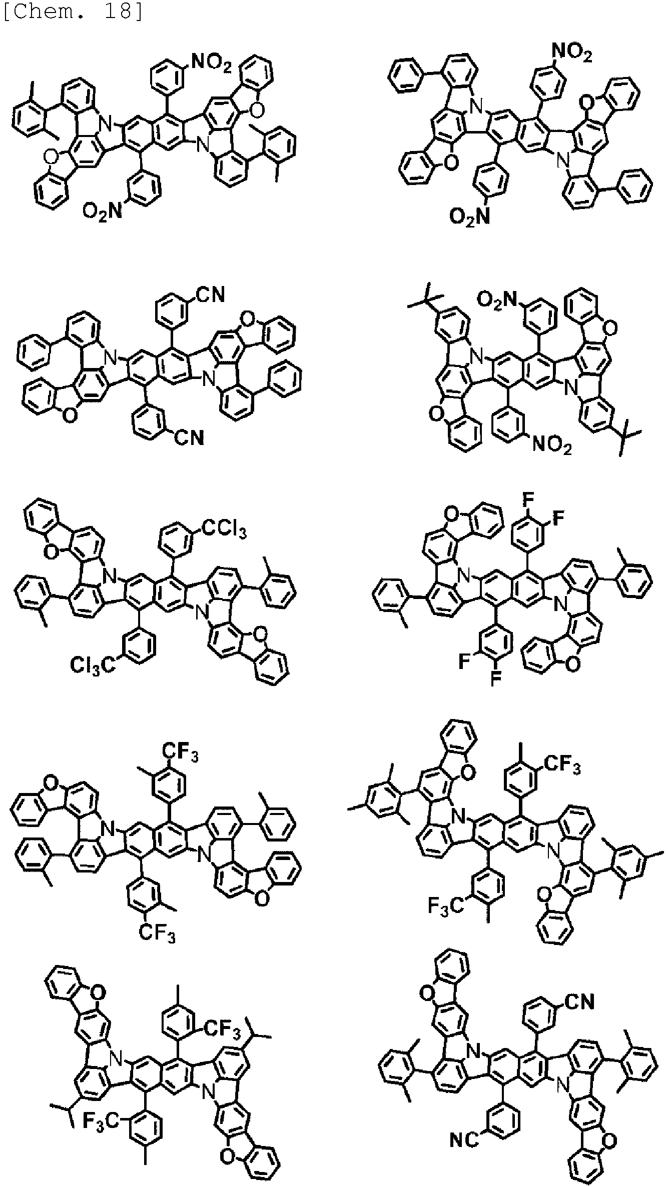

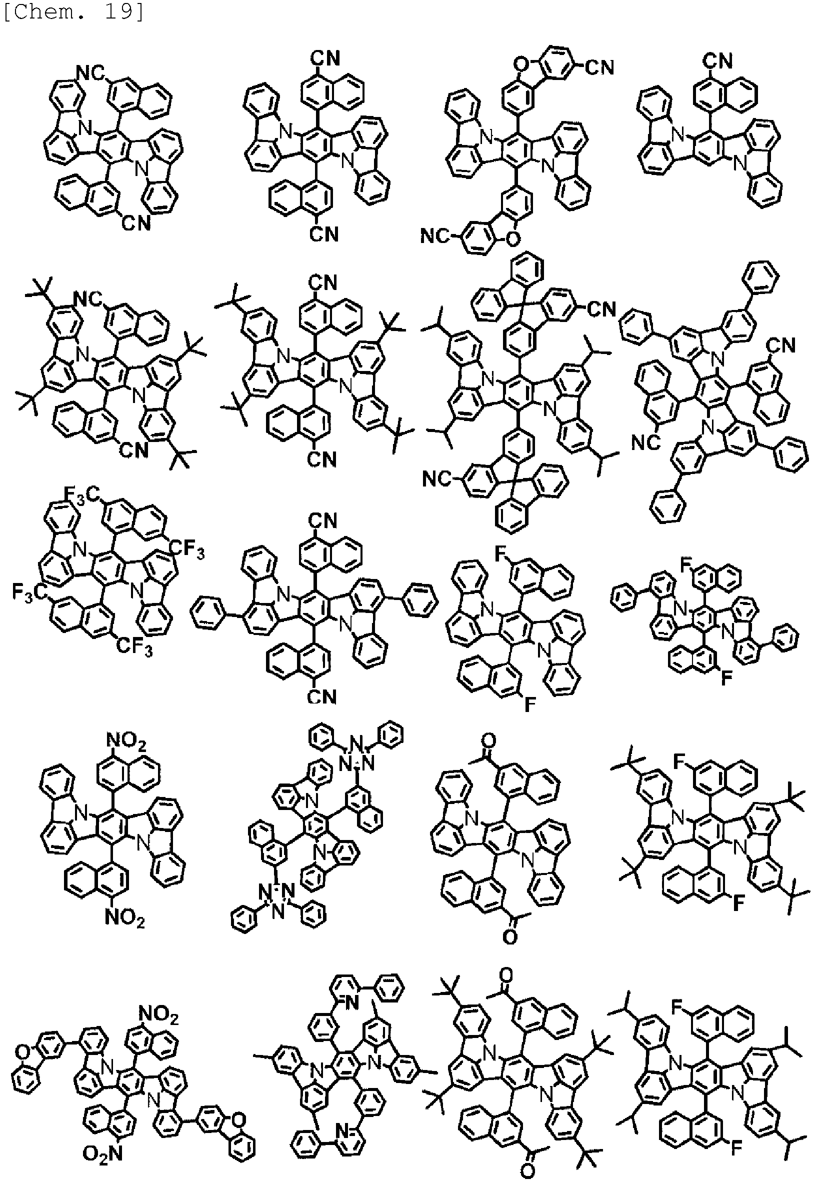

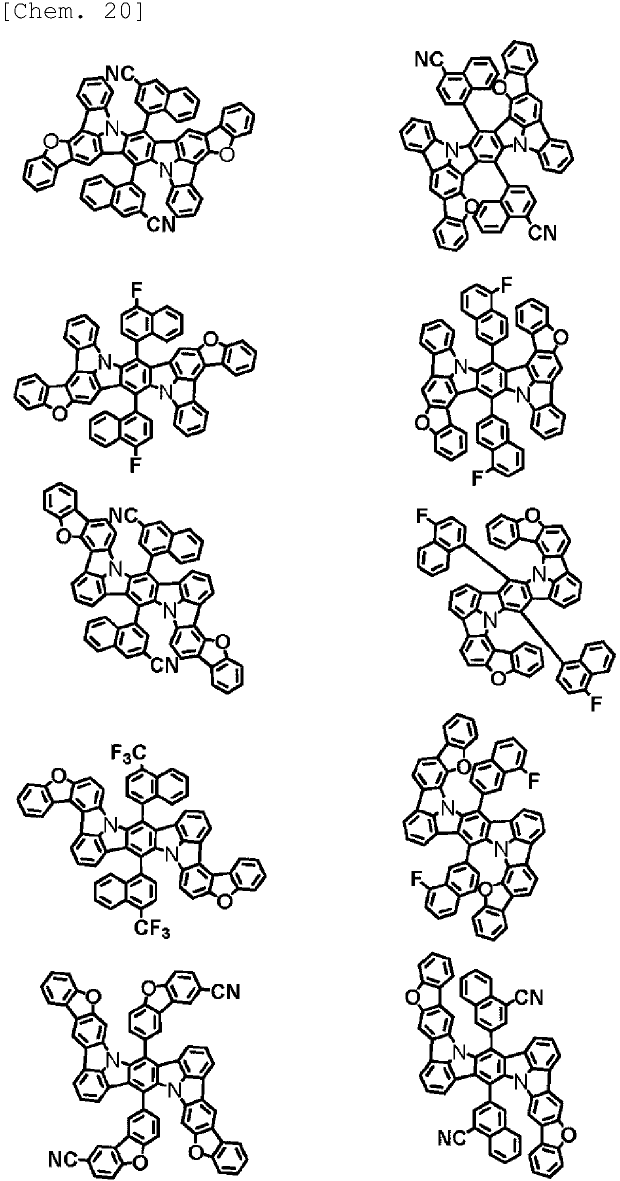

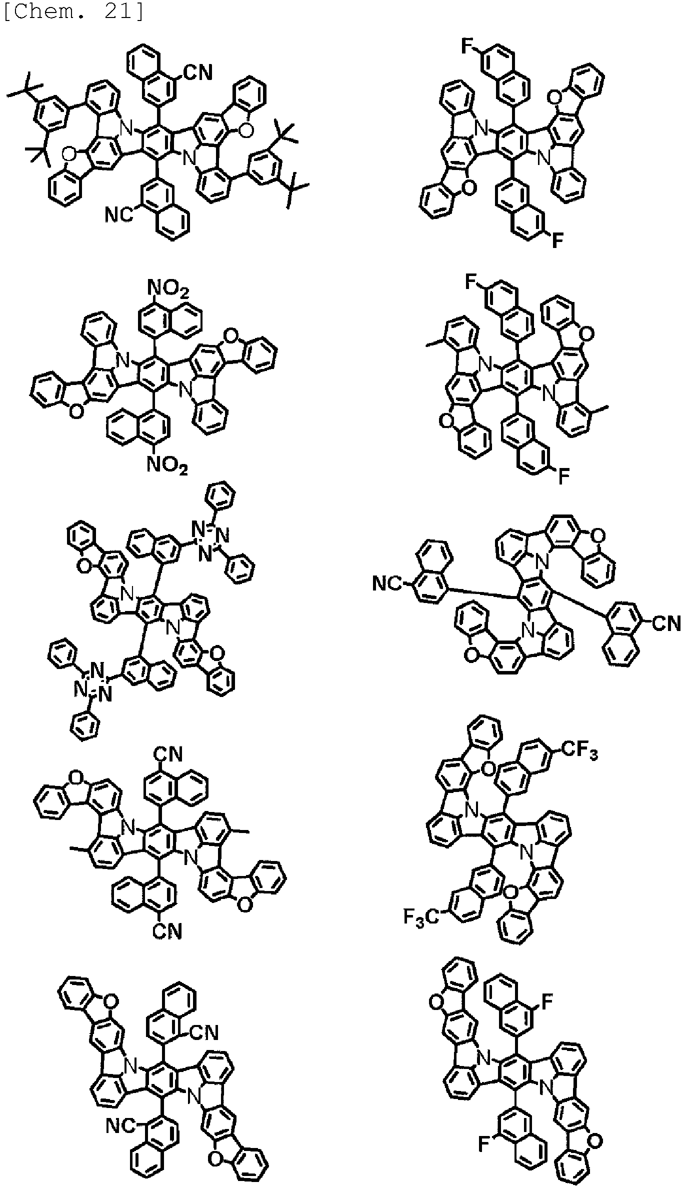

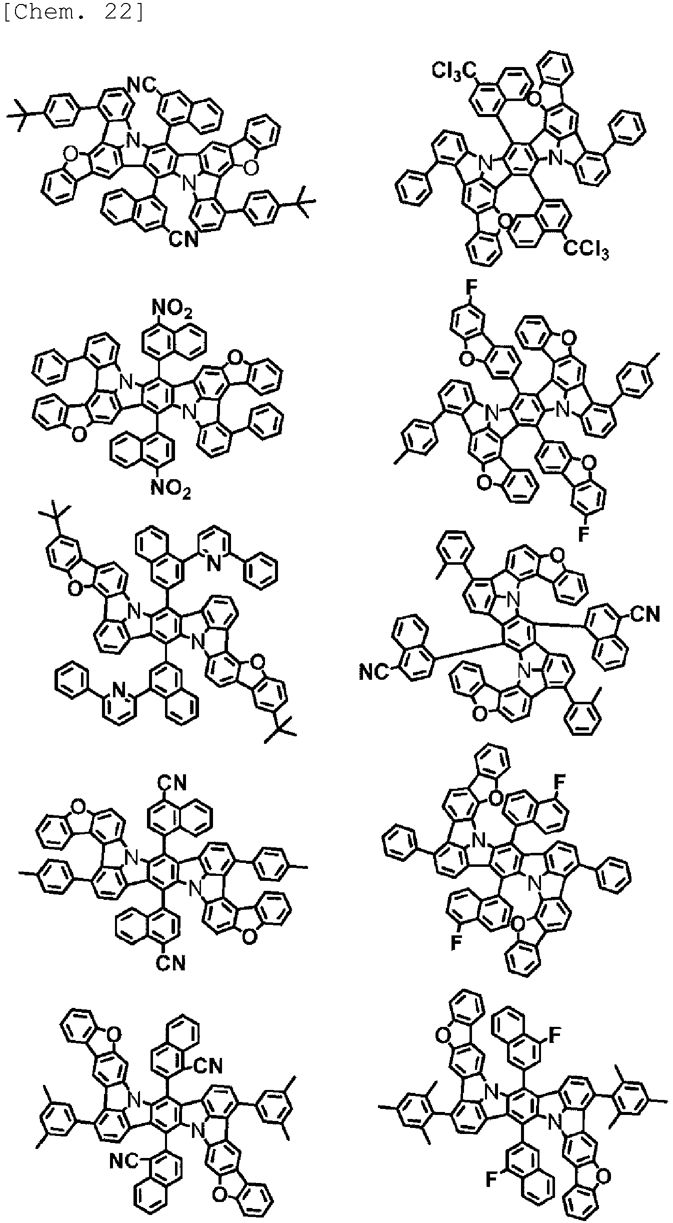

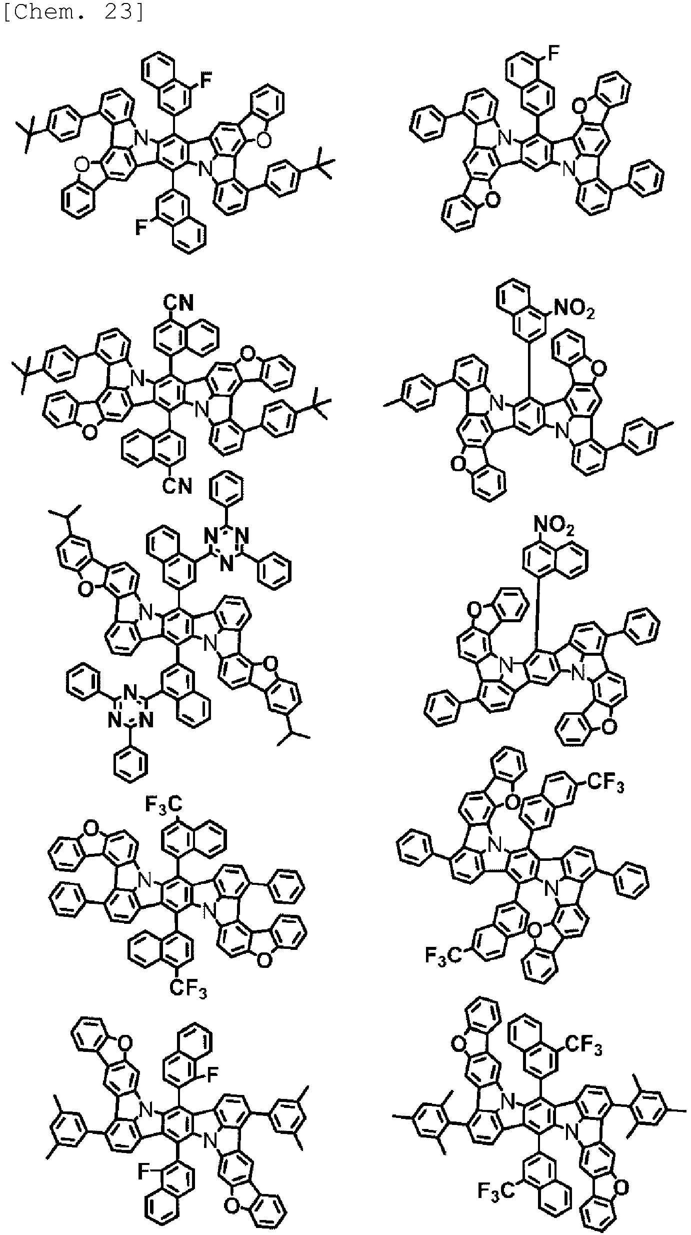

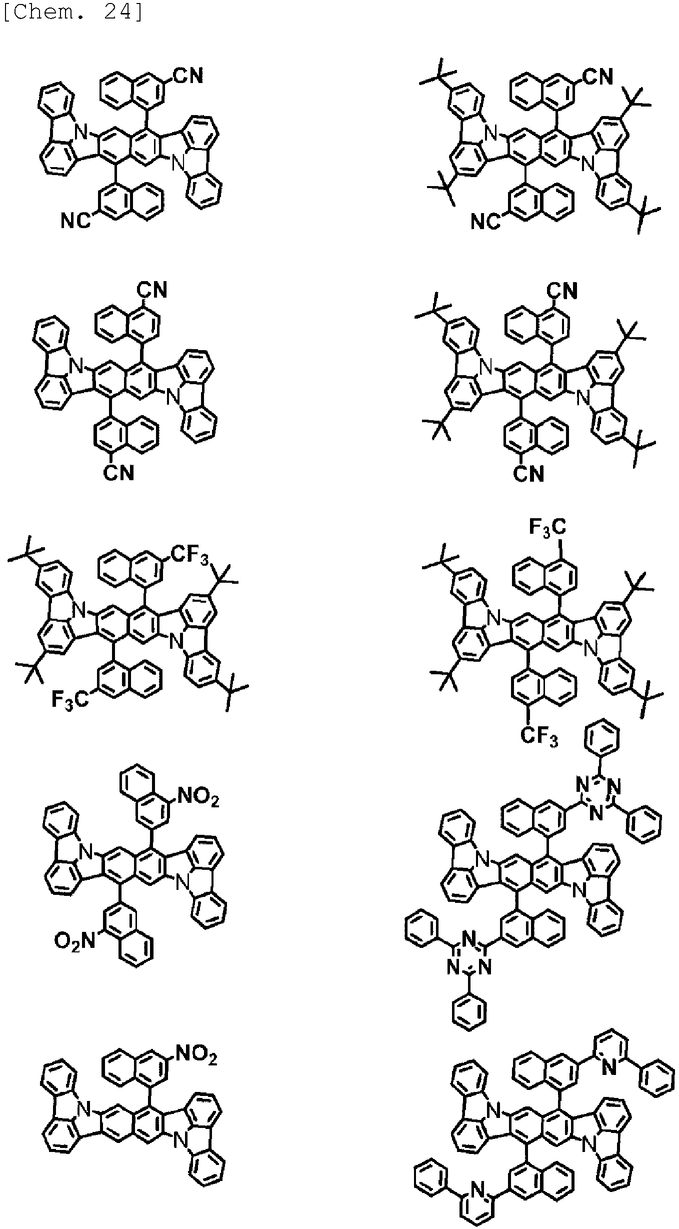

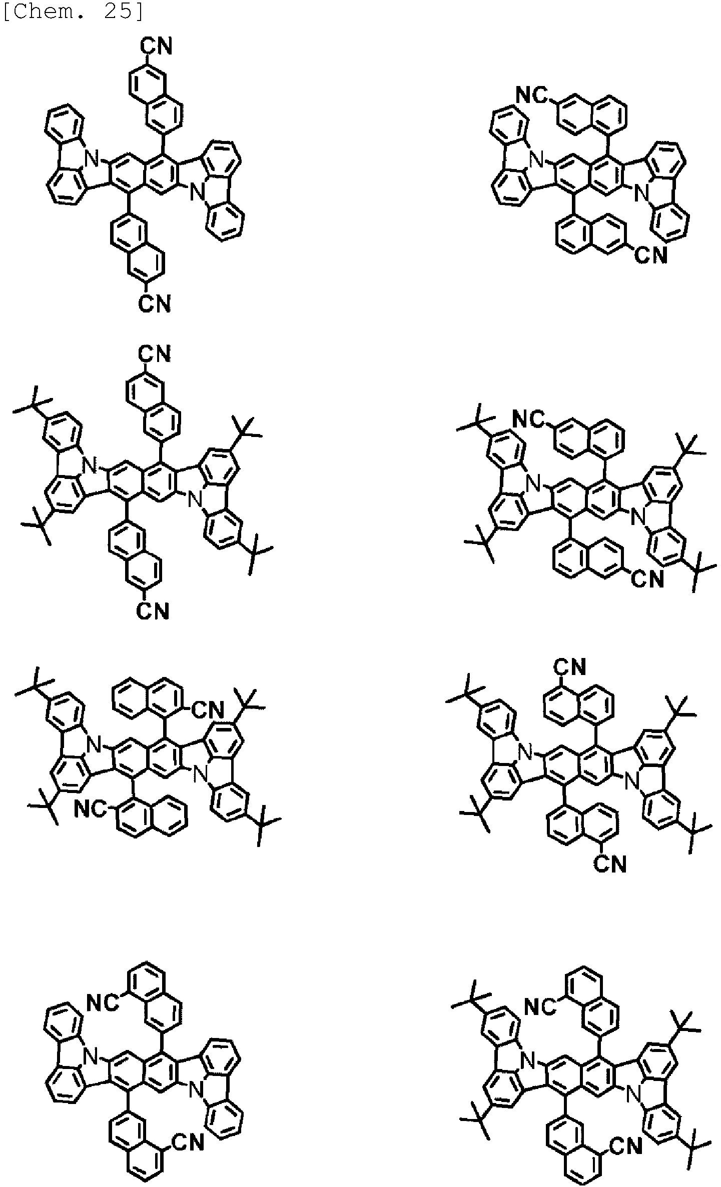

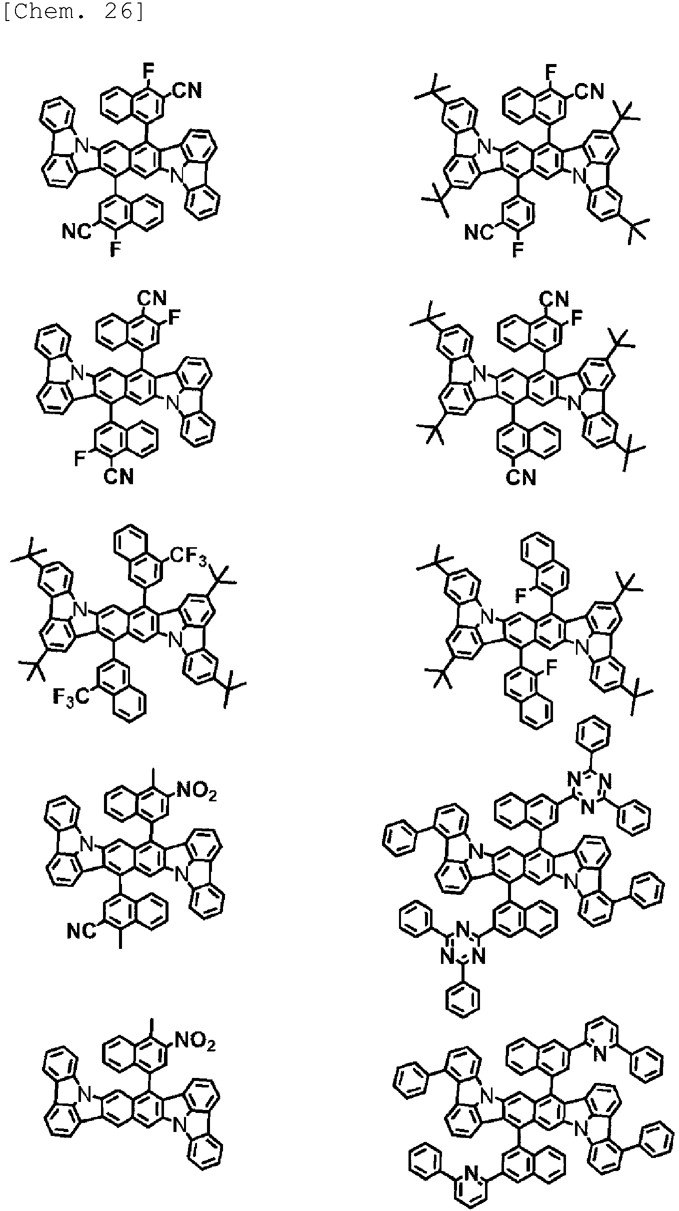

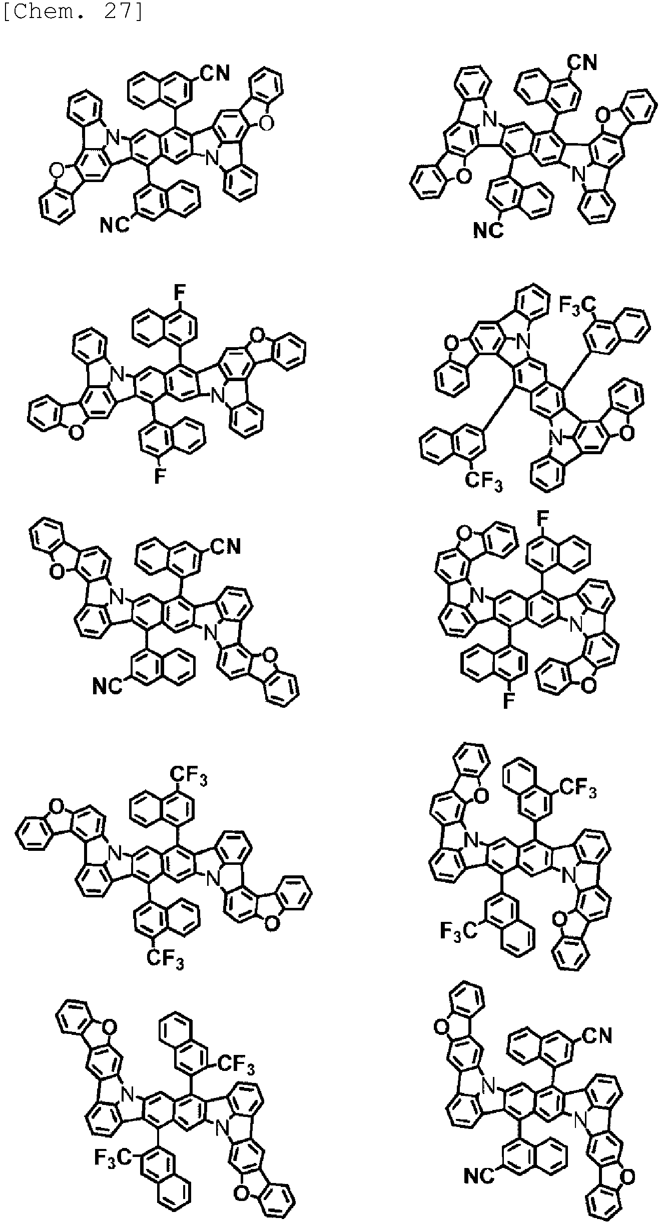

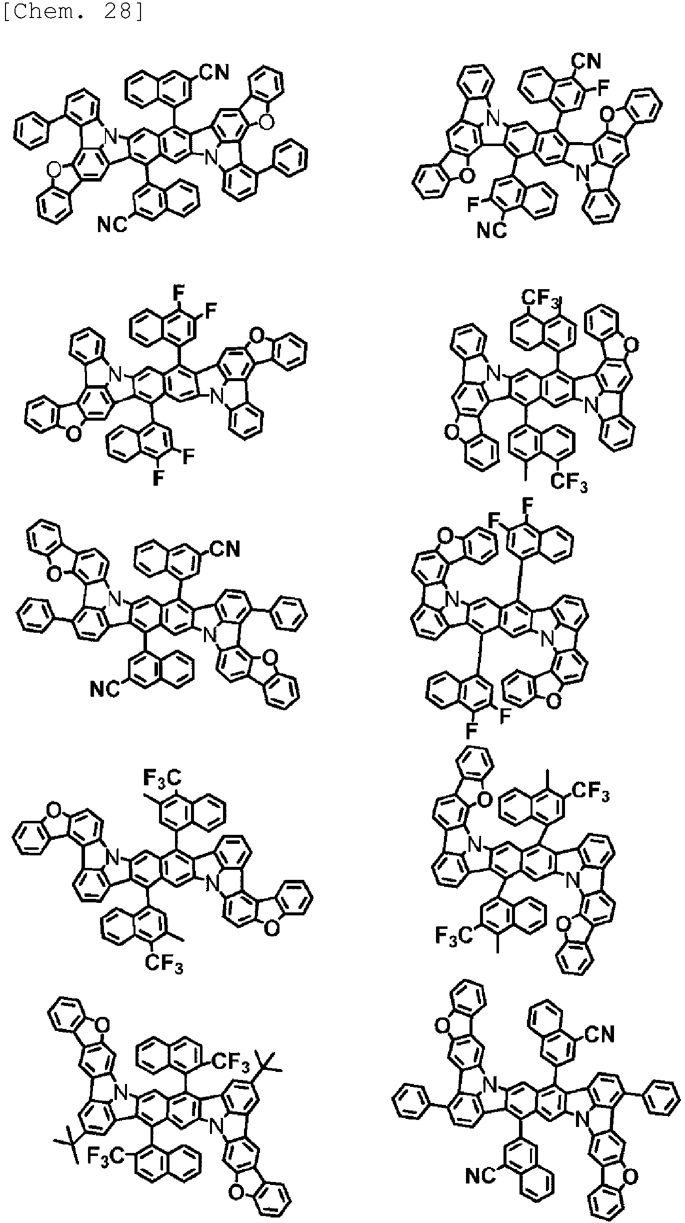

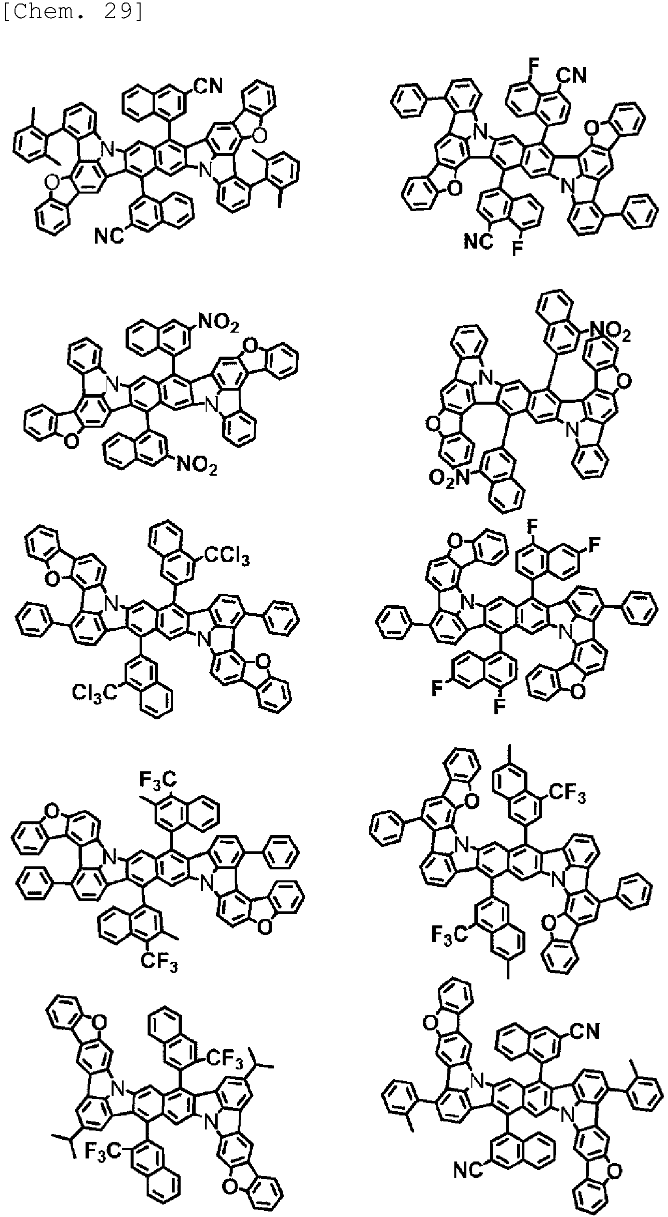

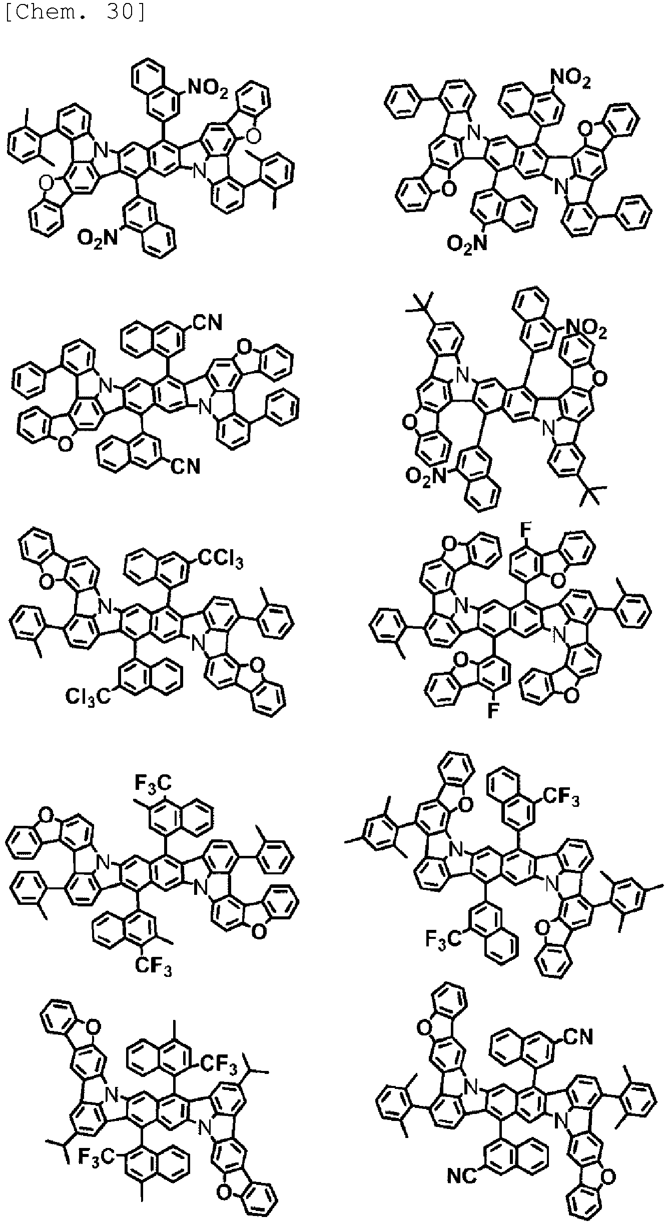

### (2) Organic Light-Emitting Device

An organic light-emitting device according to an embodiment of the present invention will be described below. The organic light-emitting device according to an embodiment of the present invention includes a first electrode, a second electrode, and an organic compound layer disposed between these electrodes. One of the first electrode and the second electrode is an anode and the other is a cathode. In the organic light-emitting device of the present embodiment, the organic compound layer may be formed of a single layer or a multilayer stack including multiple layers, as long as it includes a light-emitting layer. The organic compound according to the present invention may be contained in the organic compound layer, and is preferably contained in the light-emitting layer. When the organic compound layer is formed of a multilayer stack including multiple layers, the organic compound layer may include, in addition to the light-emitting layer, a hole (positive hole) injection layer, a hole transport layer, an electron-blocking layer, a hole/exciton-blocking layer, an electron transport layer, an electron injection layer, and so forth. The light-emitting layer may be formed of a single layer or a multilayer stack including multiple layers. In the case of providing multiple light-emitting layers, a charge generation layer may be disposed between the light-emitting layers. The charge generation layer may be made of a compound having the lowest unoccupied molecular orbital (LUMO) energy level that is lower than the LUMO energy level of the hole transport layer, and the LUMO energy level of the charge generation layer may be lower than the HOMO energy level of the hole transport layer. Here, the HOMO energy level and the LUMO energy level of the organic compound layer may be the HOMO energy level and the LUMO energy level of the organic compound having the largest ratio by weight in the organic compound layer.

Here, the HOMO energy level and the LUMO energy level are described as being "higher" as they are closer to the vacuum level. The fact that the LUMO energy level of the charge generation layer is lower than the HOMO energy level of the hole transport layer indicates that the LUMO energy level of the charge generation layer is farther from the vacuum level than the HOMO energy level of the hole transport layer.

In the present specification, the HOMO energy level and the LUMO energy level can be calculated using molecular orbital calculations. The molecular orbital calculations may be performed by density functional theory (DFT) or the like, using, for example, the B3LYP functional and the 6-31G* basis set. The molecular orbital calculations can be carried out, for example, using Gaussian 09 (Gaussian 09, Revision C.01, M. J. Frisch, G. W. Trucks, H. B. Schlegel, G. E. Scuseria, M. A. Robb, J. R. Cheeseman, G. Scalmani, V. Barone, B. Mennucci, G. A. Petersson, H. Nakatsuji, M. Caricato, X. Li, H. P. Hratchian, A. F. Izmaylov, J. Bloino, G. Zheng, J. L. Sonnenberg, M. Hada, M. Ehara, K. Toyota, R. Fukuda, J. Hasegawa, M. Ishida, T. Nakajima, Y. Honda, O. Kitao, H. Nakai, T. Vreven, J. A. Montgomery, Jr., J. E. Peralta, F. Ogliaro, M. Bearpark, J. J. Heyd, E. Brothers, K. N. Kudin, V. N. Staroverov, T. Keith, R. Kobayashi, J. Normand, K. Raghavachari, A. Rendell, J. C. Burant, S. S. Iyengar, J. Tomasi, M. Cossi, N. Rega, J. M. Millam, M. Klene, J. E. Knox, J. B. Cross, V. Bakken, C. Adamo, J. Jaramillo, R. Gomperts, R. E. Stratmann, O. Yazyev, A. J. Austin, R. Cammi, C. Pomelli, J. W. Ochterski, R. L. Martin, K. Morokuma, V. G. Zakrzewski, G. A. Voth, P. Salvador, J. J. Dannenberg, S. Dapprich, A. D. Daniels, O. Farkas, J. B. Foresman, J. V. Ortiz, J. Cioslowski, and D. J. Fox, Gaussian, Inc., Wallingford CT, 2010).

In the present specification, the HOMO energy level and the LUMO energy level can be calculated using an ionization potential and a band gap. The HOMO energy level can be estimated by measuring the ionization potential. The ionization potential can be measured with a measurement device, such as AC-3, after dissolving the compound to be measured in a solvent, such as toluene, or after forming a vapor-deposited film of the compound to be measured on a substrate, such as glass. The band gap can be measured by dissolving the compound to be measured in a solvent, such as toluene, and performing a measurement in which it is irradiated with excitation light. The band gap can be determined by measuring the absorption edge in the absorption spectrum for the excitation light. Alternatively, the measurement can be performed by vapor-depositing the compound to be measured on a substrate, such as glass, and irradiating the vapor-deposited film with excitation light. In this measurement, the band gap is determined by measuring the absorption edge in the absorption spectrum obtained when the vapor-deposited film absorbs excitation light.

The LUMO energy level can be calculated using the values of the band gap and the ionization potential. The LUMO energy level can be estimated by subtracting the ionization potential value from the band gap.

The LUMO energy level can also be estimated from the reduction potential. For example, a one-electron reduction potential is estimated using cyclic volmetry (CV) measurement. The CV measurement can be performed, for example, in a 0.1 M tetrabutylammonium perchlorate solution in DMF, using Ag/Ag⁺ serving as a reference electrode, Pt serving as a counter electrode, and glassy carbon serving as a working electrode. The LUMO energy level can be estimated by adding -4.8 eV to the difference between the reduction potential of ferrocene and the reduction potential of the obtained compound.

In the organic light-emitting device according to an embodiment of the present invention, when the organic compound according to the present invention is contained in the light-emitting layer, the light-emitting layer may be a layer made only of the organic compound according to the present invention, or may be a layer made of the organic compound according to the present invention and another compound. Here, when the light-emitting layer is a layer made of the organic compound according to the present invention and another compound, the organic compound according to the present invention may be used as a host material in the light-emitting layer, may be used as a guest material, or may be used as an assist material that can be contained in the light-emitting layer. Here, the host material is also referred to as a "host" or a "first compound" and is a compound having the largest mass ratio among the compounds constituting the light-emitting layer. The guest material is also referred to as a "guest", a "dopant material", a "dopant" or a "third compound". The guest material is a compound having a smaller mass ratio than the host among the compounds constituting the light-emitting layer and is a compound that mainly contributes to light emission. Therefore, the guest material is sometimes also called a light-emitting material. The assist material is also referred to as an "assist" or a "second compound". The assist material is a compound that has a smaller mass ratio than the host material among the compounds constituting the light-emitting layer and that assists light emission of the guest material. The assist material is also called a second host.

Here, the lowest excited singlet energy of the host material is denoted as S1(H), the lowest excited singlet energy of the guest material is denoted as S1(D), and the lowest excited singlet energy of the assist material is denoted as S1(A). The guest material may be regarded as the organic compound according to the present invention. In this case, the organic light-emitting device according to the present embodiment preferably satisfies S1(H) > S1(D) or S1(H) > S1(A) > S1(D). When the lowest excited singlet energies of the compounds contained in the organic light-emitting device according to the present embodiment satisfy the above relationship, excitons can be efficiently transferred to the guest material, thereby providing an organic light-emitting device having higher luminous efficiency.

When the organic compound according to the present invention is used as a guest material in the light-emitting layer, the concentration of the guest material may be 0.01% or more by mass and less than 50% by mass, and is preferably 0.01% or more by mass and 10% or less by mass, more preferably 0.01% or more by mass and 5% or less by mass, relative to the light-emitting layer as a whole.

When the light-emitting layer further contains an assist material, the assist material may be 1% or more by mass and less than 50% by mass, and is preferably 10% or more by mass and less than 50% by mass, relative to the light-emitting layer as a whole. The guest may be 0.01% or more by mass and 20% or less by mass, and is preferably 0.01% or more by mass and 5% or less by mass.

The inventors have conducted various studies and have found that, when the organic compound according to the present invention is used as a host material or a guest material in a light-emitting layer, and particularly when it is used as a guest material in a light-emitting layer, a device that exhibits light output with high efficiency and high luminance and has extremely high durability can be obtained. The light-emitting layer may be a single layer or may include multiple light-emitting layers, and it is also possible to mix the blue emission, which is the emission color of the present embodiment, with emission from another light-emitting material that emits a different color by containing such another light-emitting material. The term "multiple light-emitting layers" means a state in which the light-emitting layer and another light-emitting layer are stacked. In this case, the emission color of the organic light-emitting device is not limited to blue. More specifically, the emission color may be white or an intermediate color. In the case of white, the other light-emitting layer emits a color other than blue, that is, red or green. In addition, a film may be formed by a film-forming method, such as vapor deposition or coating. Details thereof will be described in detail in Examples described later.

The organic compound according to the present invention can be used as a constituent material of an organic compound layer other than the light-emitting layer constituting the organic light-emitting device according to the present embodiment. Specifically, the organic compound may be used as a constituent material of an electron transport layer, an electron injection layer, a hole transport layer, a hole injection layer, a hole-blocking layer, or the like. In this case, the emission color of the organic light-emitting device is not limited to blue. More specifically, the emission color may be white emission or an intermediate color.

### (3) Other Compounds

Here, in addition to the organic compound according to the present invention, conventionally known low-molecular-weight and high-molecular-weight hole-injecting compounds or hole-transporting compounds, host materials, light-emitting compounds, electron-injecting compounds or electron-transporting compounds, and the like may be used together, as needed. Examples of these compounds are described below.

As a hole injection/transport material, a material that facilitates injection of holes from the anode and has high hole mobility so that the injected holes can be transported to the light-emitting layer is preferable. In order to inhibit crystallization of the organic compound and the like in the organic light-emitting device, a material having a high glass transition temperature is preferable. Examples of low-molecular-weight and high-molecular-weight materials having the ability to inject/transport holes include triarylamine derivatives, arylcarbazole derivatives, phenylenediamine derivatives, stilbene derivatives, phthalocyanine derivatives, porphyrin derivatives, poly(vinylcarbazole), poly(thiophene), and other conductive polymers. The above hole injection/transport materials are also suitably used for an electron-blocking layer. When a hole injection layer is formed by a coating method, a mixture (PEDOT:PSS) of poly(ethylenedioxythiophene) and poly(styrenesulfonate), which is generally used as a hole injection material, may be used. Specific examples of compounds used as hole injection/transport materials are illustrated below, but, of course, the compounds are not limited thereto.

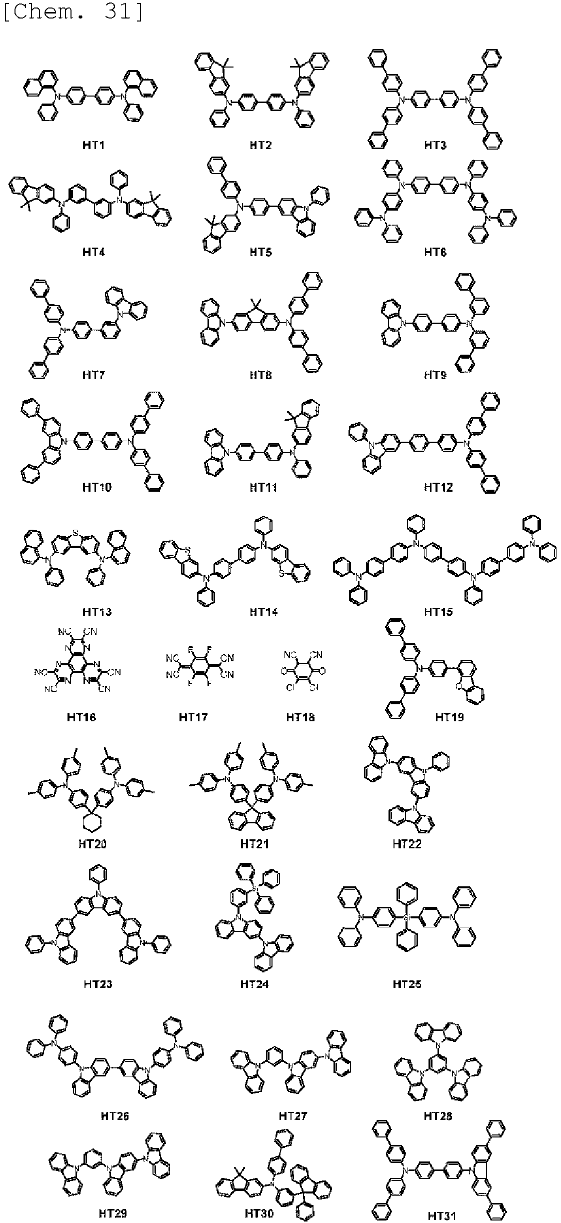

Among the hole injection/transport materials described above, HT16 to HT18 can reduce the driving voltage when used in a layer in contact with the anode. HT16 is widely used in organic light-emitting devices. HT2 to HT7, HT10, HT12, and HT22 to HT28 may be used in an organic compound layer adjacent to HT16. Hole-transporting polymer compounds, such as poly(phenylene vinylene) (PPV), polyfluorene (PF), polyvinylcarpazole (PVK), and derivatives thereof may also be used. In addition, an inorganic insulating layer made of, for example, SiO2 or SiN, or an organosilicon polymer, such as siloxane, may also be used. Furthermore, a plurality of materials may be used in one organic compound layer.

Examples of light-emitting materials mainly involved in the light-emitting function include donor-acceptor type organic compounds, boron-containing complexes, indocarbazole fused-ring compounds, and fused-ring compounds (such as fluorene derivatives, naphthalene derivatives, pyrene derivatives, perylene derivatives, tetracene derivatives, anthracene derivatives, and rubrene), quinacridone derivatives, coumarin derivatives, stilbene derivatives, organoaluminum complexes, such as tris(8-quinolinolato)aluminum, iridium complexes, platinum complexes, rhenium complexes, copper complexes, europium complexes, ruthenium complexes, and polymer derivatives, such as poly(phenylene vinylene) derivatives, poly(fluorene) derivatives, and poly(phenylene) derivatives. In addition, when a light-emitting layer is formed by a coating method, polymer compounds having light-emitting properties are mainly used. This is because polymer compounds are highly amorphous and are less likely to crystallize than low-molecular-weight compounds. Specific examples of a material used include polymer compounds, such as poly(phenylene vinylene) (PPV), polyfluorene (PF), polyvinylcarpazole (PVK), and derivatives thereof.

Specific examples of a compound that can be used as a light-emitting material are illustrated below, but, of course, the compound is not limited thereto.

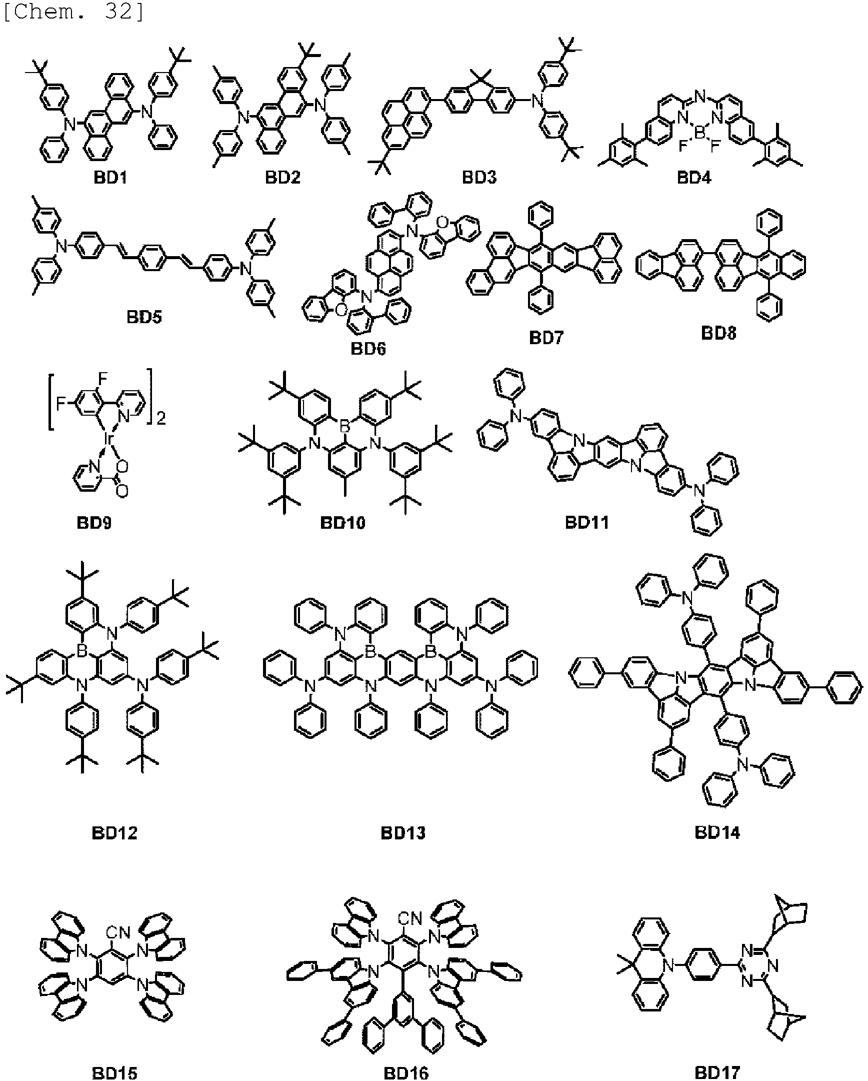

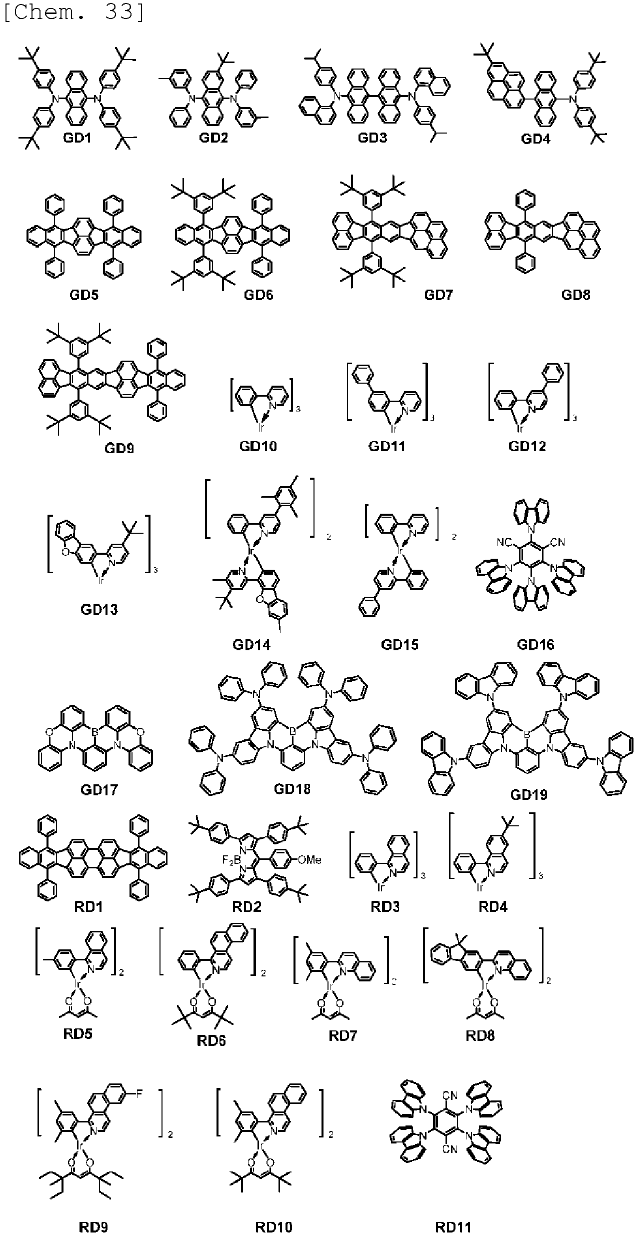

Specific examples of a compound used as a host in the light-emitting layer or as an emission-assist dopant contained in the light-emitting layer are illustrated below, but, of course, the compound is not limited thereto.

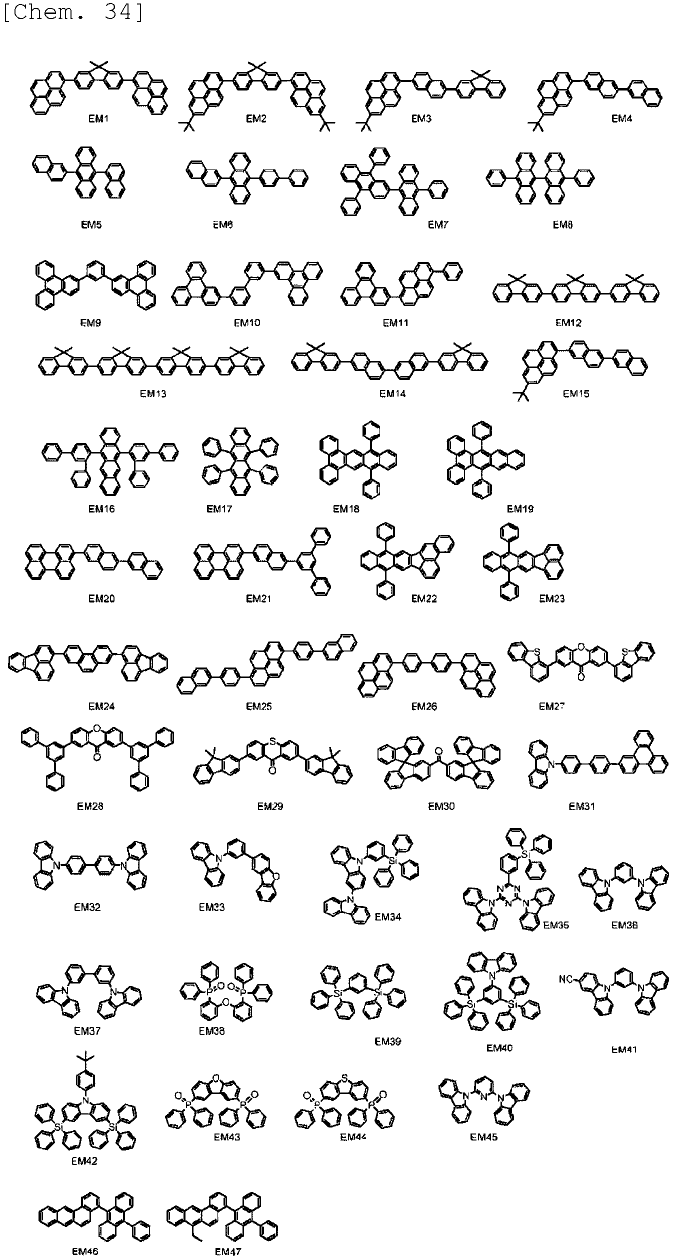

Among EM1 to EM47, the host material may be a hydrocarbon compound having a fused polycyclic hydrocarbon group. Specific examples include EM1 to EM26, EM46, and EM47.

As the electron-transporting material, any material that can transport electrons injected from the cathode to the light-emitting layer can be selected, and the selection is made in consideration of factors, such as the balance with the hole mobility of the hole-transporting material. Examples of materials having the ability to transport electrons include oxadiazole derivatives, oxazole derivatives, pyrazine derivatives, triazole derivatives, triazine derivatives, quinoline derivatives, quinoxaline derivatives, phenanthroline derivatives, organoaluminum complexes, and fused-ring compounds (such as fluorene derivatives, naphthalene derivatives, chrysene derivatives, and anthracene derivatives). The above electron-transporting materials are also suitably used in a hole-blocking layer.

Specific examples of a compound used as an electron-transporting material are given below, but, of course, the compound is not limited thereto.

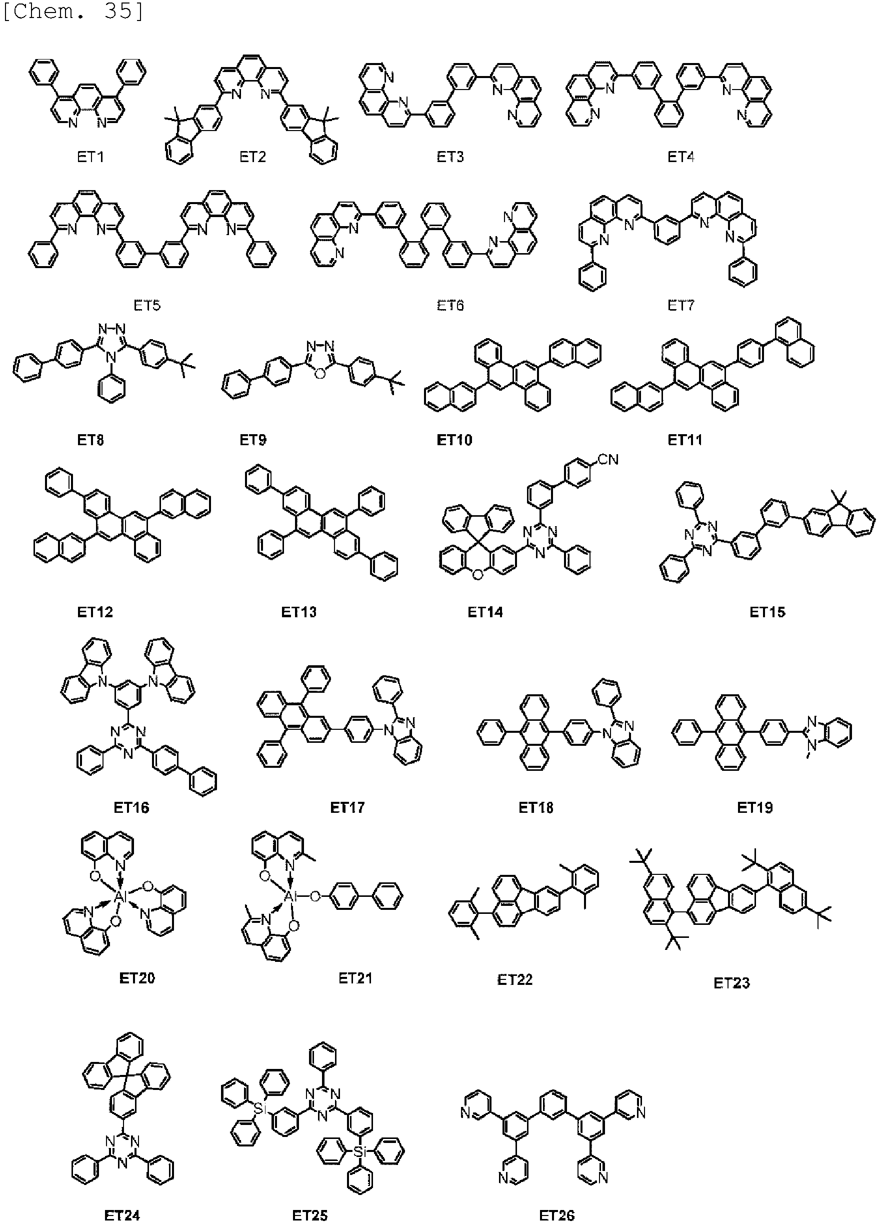

An electron-injecting material can be freely selected from materials into which electrons can be easily injected from the cathode and is selected in consideration of, for example, the balance with the hole-injection properties. As the organic compound, n-type dopants and reducing dopants are also included. Examples thereof include alkali metal-containing compounds, such as lithium fluoride, lithium complexes, such as lithium quinolinolate, benzimidazolidene derivatives, imidazolidene derivatives, fulvalene derivatives, and acridine derivatives.

It can also be used in combination with the above electron-transporting material.

### (4) Configuration of Organic Light-Emitting Device

Constituent members constituting the organic light-emitting device of the present embodiment will be described below.

The organic light-emitting device includes, over the substrate, an insulating layer, the first electrode, the organic compound layer, and the second electrode. A protective layer, a color filter, a microlens, and so forth may be disposed over the second electrode. When a color filter is provided, a planarization layer may be provided between the color filter and the protective layer. The planarization layer can be made of an acrylic resin or the like. The same applies when a planarization layer is provided between the color filter and the microlens.

### [Substrate]

Examples of the substrate include quartz, glass, silicon wafers, resins, and metals. The substrate may include a switching element, such as a transistor, a line, and an insulating layer thereon. Any material can be used for the insulating layer as long as a contact hole can be formed in such a manner that a line can be coupled to the first electrode and as long as insulation with a non-connected line can be ensured. For example, a resin, such as polyimide, silicon oxide, or silicon nitride, can be used.

### [Electrodes]

As the electrodes, a pair of electrodes can be used. The pair of electrodes is the first electrode and the second electrode. Specifically, the pair of electrodes may be the anode and the cathode. When an electric field is applied in the direction in which the organic light-emitting device emits light, an electrode having a higher potential is the anode, and the other is the cathode. It can also be said that the electrode that supplies holes to the light-emitting layers is the anode and that the electrode that supplies electrons is the cathode.

It is preferable for the material constituting the anode to have a work function that is as high as possible. Examples of the material that can be used include elemental metals, such as gold, platinum, silver, copper, nickel, palladium, cobalt, selenium, vanadium, and tungsten, mixtures thereof, alloys of combinations thereof, and metal oxides, such as tin oxide, zinc oxide, indium oxide, indium-tin oxide (ITO), and indium-zinc oxide. Additionally, conductive polymers, such as polyaniline, polypyrrole, and polythiophene, can also be used.

These electrode materials may be used alone or in combination of two or more. The anode may be formed of a single layer or multiple layers.

When the electrode is used as a reflective electrode, for example, chromium, aluminum, silver, titanium, tungsten, molybdenum, an alloy thereof, or a stack thereof can be used. These materials can also be used to act as a reflective film that does not have the role of an electrode. When the electrode is used as a transparent electrode, a transparent conductive oxide layer made of, for example, indium-tin oxide (ITO) or indium-zinc oxide can be used; however, the electrode is not limited thereto. The electrode can be formed by a photolithography technique.

As a constituent material of the cathode, a material having a small work function is preferred. Examples thereof include elemental metals, such as alkali metals, e.g., lithium, alkaline-earth metals, e.g., calcium, aluminum, titanium, manganese, silver, lead, and chromium, and mixtures containing them. Alternatively, an alloy made by combining these elemental metals can also be used. For example, magnesium-silver, aluminum-lithium, aluminum-magnesium, silver-copper, zinc-silver, and so forth can be used. Metal oxides, such as indium-tin oxide (ITO), can also be used. These electrode materials may be used alone or in combination of two or more. The cathode may have a single-layer structure or a multilayer structure. In particular, silver is preferably used. To reduce the aggregation of silver, a silver alloy is more preferred. Any alloy ratio may be used as long as the aggregation of silver can be reduced. The ratio of silver to another metal may be, for example, 1:1 or 3:1.

A top-emission device may be provided using the cathode formed of a conductive oxide layer composed of, for example, ITO. A bottom-emission device may be provided using the cathode formed of a reflective electrode made of, for example, aluminum (Al). The cathode is not particularly limited. A method for forming the cathode is not particularly limited, but a direct-current sputtering method, an alternating-current sputtering method, or the like is more preferably used because good film coverage is obtained and thus the resistance is easily reduced.

### [Organic Compound Layer]

The organic compound layer may be formed of a single layer or multiple layers. When multiple layers are present, they may be referred to as a hole injection layer, a hole transport layer, an electron-blocking layer, a light-emitting layer, a hole-blocking layer, an electron transport layer, or an electron injection layer in accordance with their functions. The organic compound layer is mainly made of an organic compound, and may contain inorganic atoms and an inorganic compound. For example, each organic compound layer may contain, for example, copper, lithium, magnesium, aluminum, iridium, platinum, molybdenum, or zinc. The organic compound layer may be disposed between the first electrode and the second electrode, and may be disposed in contact with the first electrode and the second electrode.

The organic compound layer (such as the hole injection layer, the hole transport layer, the electron-blocking layer, the light-emitting layer, the hole-blocking layer, the electron transport layer, or the electron injection layer) included in the organic light-emitting device according to the present embodiment is formed by a method described below.

For the organic compound layer included in the organic light-emitting device according to the present embodiment, a dry process, such as a vacuum evaporation method, an ionized evaporation method, sputtering, or a plasma process, can be employed. Alternatively, instead of the dry process, it is also possible to employ a wet process in which a material is dissolved in an appropriate solvent and then a film is formed by a known coating method (for example, spin coating, dipping, a casting method, an LB technique, or an ink jet method).

When the layer is formed by, for example, the vacuum evaporation method or the solution coating method, crystallization and so forth are less likely to occur, and excellent stability with time is obtained. In the case of forming a film by the coating method, the film can also be formed in combination with an appropriate binder resin.

Examples of the binder resin include, but are not limited to, polyvinylcarbazole resins, polycarbonate resins, polyester resins, ABS resins, acrylic resins, polyimide resins, phenolic resins, epoxy resins, silicon resins, and urea resins.

These binder resins may be used alone as a homopolymer or copolymer or in combination as a mixture of two or more. Furthermore, additives, such as a known plasticizer, antioxidant, and ultraviolet absorber, may be used, as needed.

### [Protective Layer]

A protective layer may be disposed on the cathode. For example, glass provided with a moisture absorbent can be bonded to the cathode to reduce the entry of, for example, water into the organic compound layer, thereby reducing the occurrence of display defects. In another embodiment, a passivation film made of, for example, silicon nitride may be disposed on the cathode to reduce the entry of, for example, water into the organic compound layer. For example, after the formation of the cathode, the substrate may be conveyed to another chamber without breaking the vacuum, and a silicon nitride film having a thickness of 2 µm may be formed by a CVD method to form a protective layer. After the film deposition by the CVD method, a protective layer may be formed by an atomic layer deposition method (ALD method). Examples of the material of the layer formed by the ALD method may include, but are not limited to, silicon nitride, silicon oxide, and aluminum oxide. Silicon nitride may be deposited by the CVD method on the layer formed by the ALD method. The film formed by the ALD method may have a smaller thickness than the film formed by the CVD method. Specifically, the thickness of the film formed by the ALD method may be 50% or less, or even 10% or less, of the thickness of the film formed by the CVD method.

### [Color Filter]

A color filter may be disposed on the protective layer. For example, a color filter may be disposed on another substrate in consideration of the size of the organic light-emitting device and bonded to the substrate provided with the organic light-emitting device. A color filter may be formed by patterning on the protective layer using a photolithography technique. The color filter may be made of a polymer.

### [Planarization Layer]

A planarization layer may be disposed between the color filter and the protective layer. The planarization layer is provided for the purpose of reducing the unevenness of the underlying layer. The planarization layer may be referred to as a material resin layer without limiting its purpose. The planarization layer may be made of an organic compound, may have a low-molecular-weight or high-molecular-weight compound, and is preferably a high-molecular-weight compound.

The planarization layers may be disposed above and below the color filter and may be composed of the same or different constituent materials. Specific examples thereof include polyvinylcarbazole resins, polycarbonate resins, polyester resins, ABS resins, acrylic resins, polyimide resins, phenolic resins, epoxy resins, silicone resins, and urea resins.

### [Microlens]

The organic light-emitting device according to the present embodiment may include an optical member, such as a microlens, on the light-emitting side. The microlens can be made of acrylic resin, epoxy resin, or the like. The microlens may be used to increase the amount of light emitted from the organic light-emitting device and to control the direction of the light emitted. The microlens may have a hemispherical shape. In the case of a hemispherical shape, among tangents to the hemisphere, there is a tangent parallel to the insulating layer. The point of contact of the tangent with the hemisphere is the vertex of the microlens. The vertex of the microlens can be determined in the same way for any cross-sectional view. That is, among the tangents to the semicircle of the microlens in the cross-sectional view, there is a tangent parallel to the insulating layer, and the point of contact of the tangent with the semicircle is the vertex of the microlens.

The midpoint of the microlens can also be defined. In the cross section of the microlens, when a segment is hypothetically drawn from a point at which an arc shape begins to a point at which another arc shape begins, the midpoint of the segment can be referred to as the midpoint of the microlens. The cross section to determine the vertex and midpoint may be a cross section perpendicular to the insulating layer.

### [Opposite Substrate]

An opposite substrate may be disposed on the planarization layer. The opposite substrate is disposed at a position corresponding to the substrate described above and thus is called an opposite substrate. The opposite substrate may be made of the same constituent material as that of the substrate described above. When the above-described substrate is referred to as a first substrate, the opposite substrate may be referred to as a second substrate.

### [Pixel Circuit]

A light-emitting apparatus may include pixel circuits coupled to organic light-emitting devices. The pixel circuit may be of an active matrix type that independently controls the light emission of a first light-emitting device and a second light-emitting device. The active matrix-type circuit may be voltage-programmed or current-programmed. A driving circuit includes the pixel circuit for each pixel. The pixel circuit may include a light-emitting device, a transistor to control the luminance of emission light from the light-emitting device, a transistor to control the timing of the light emission, a capacitor to retain the gate voltage of the transistor to control the luminance of emission light, and a transistor to connect to GND without using the light-emitting device.

The light-emitting apparatus includes a display area and a peripheral area disposed around the display area. The display area includes a pixel circuit, and the peripheral area includes a display control circuit. The mobility of a transistor contained in the pixel circuit may be lower than the mobility of a transistor contained in the display control circuit.

The slope of the current-voltage characteristics of the transistor contained in the pixel circuit may be smaller than the slope of the current-voltage characteristic of the transistor contained in the display control circuit. The slope of the current-voltage characteristics can be measured by what is called Vg-Ig characteristics.

The transistor contained in the pixel circuit is a transistor coupled to a light-emitting device, such as the first light-emitting device.

### [Pixel]

The organic light-emitting apparatus includes multiple pixels. Each pixel includes subpixels configured to emit colors different from each other. The subpixels may have respective RGB emission colors.

Light emerges from a region of the pixel, also called a pixel aperture. This region is the same as a first region. The pixel aperture may be 15 µm or less, and may be 5 µm or more. More specifically, the pixel aperture may be, for example, 11 µm, 9.5 µm, 7.4 µm, or 6.4 µm.

The distance between subpixels may be 10 µm or less. Specifically, the distance may be 8 µm, 7.4 µm, or 6.4 µm.

The pixels may be arranged in a known configuration in plan view. For example, a stripe pattern, a delta pattern, a Pen Tile matrix pattern, or the Bayer pattern may be used. The shape of each subpixel in plan view may be any known shape. Examples thereof include quadrilaterals, such as rectangles and rhombi, and hexagons. Of course, if the shape is close to a rectangle, rather than an exact shape, it is included in the rectangle. The shape of the subpixel and the pixel arrangement can be used in combination.

### (5) Application of Organic Light-Emitting Device According to Present Embodiment

The organic light-emitting device according to the present embodiment can be used as a component member of a display apparatus or lighting apparatus. Other applications include exposure light sources for electrophotographic image-forming apparatuses, backlights for liquid crystal display apparatuses, and light-emitting apparatuses including white light sources provided with color filters.

The display apparatus may be an image information-processing apparatus including an image input unit that receives image information from an area CCD, a linear CCD, a memory card, or the like, an information-processing unit that processes the input information, and a display unit that displays the input image.

The display unit of an image pickup apparatus or an ink jet printer may have a touch panel function. The driving method for the touch panel function may be, but is not particularly limited to, an infrared method, an electrostatic capacitive method, a resistive-film method, or an electromagnetic inductive method. The display apparatus may also be used for a display unit of a multifunction printer.

The following describes a display apparatus according to the present embodiment with reference to the drawings.

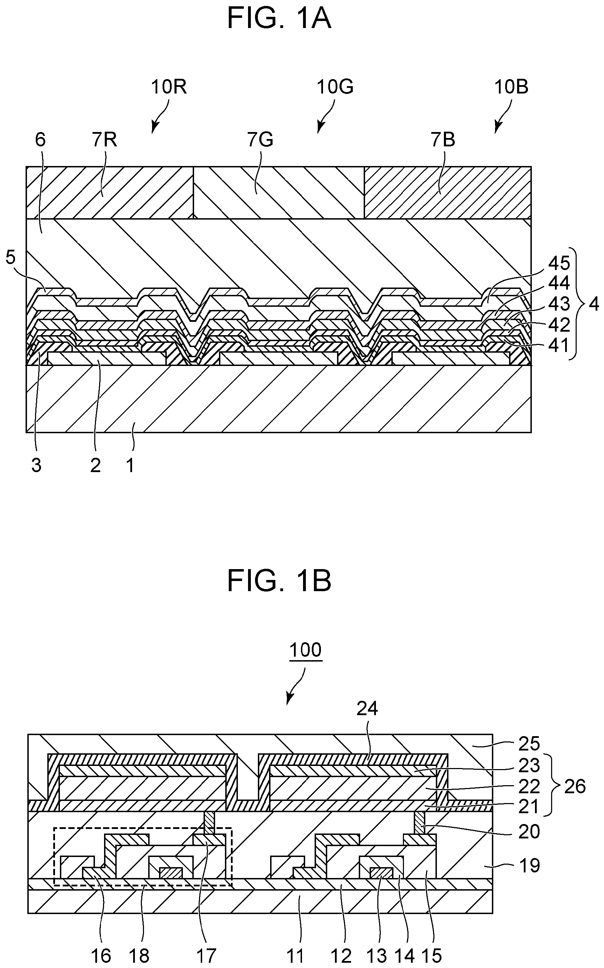

Figs. 1A and 1B are each a schematic cross-sectional view illustrating an example of a display apparatus including organic light-emitting devices and transistors coupled to the organic light-emitting devices. Each of the transistors is an example of an active element. The transistors may be thin-film transistors (TFTs).

Fig. 1A illustrates an example of a pixel that is a constituent element of the display apparatus according to the present embodiment. The pixel includes subpixels 10. The subpixels are divided into 10R, 10G, and 10B according to their light emission. The emission colors may be distinguished by the wavelength of light emitted from the light-emitting layer. Light emitted from the subpixels may be selectively transmitted or color-converted with, for example, a color filter. Each of the subpixels includes a reflective electrode 2 serving as a first electrode, an insulating layer 3 covering the edge of the reflective electrode 2, an organic compound layer 4 covering the first electrode and the insulating layer, a transparent electrode 5, a protective layer 6, and a color filter 7, over an interlayer insulating layer 1.

The transistors and capacitive elements may be disposed under or in the interlayer insulating layer 1. Each transistor may be electrically coupled to a corresponding one of the first electrodes through, for example, a contact hole (not illustrated).

The insulating layer 3 is also referred to as a bank or pixel separation film. The insulating layer covers the edge of each first electrode and surrounds the first electrode. Portions that are not covered with the insulating layer are in contact with the organic compound layer 4 and serve as light-emitting regions.

The organic compound layer 4 includes a hole injection layer 41, a hole transport layer 42, a first light-emitting layer 43, a second light-emitting layer 44, and an electron transport layer 45.

The second electrode 5 may be a transparent electrode, a reflective electrode, or a semi-transparent electrode.

The protective layer 6 reduces the penetration of moisture into the organic compound layer. Although the protective layer is illustrated as a single layer, the protective layer may be formed of multiple layers. Each layer may be an inorganic compound layer or an organic compound layer.

The color filter 7 is separated into 7R, 7G, and 7B according to its color. The color filter may be disposed on a planarization film, which is not illustrated. A resin protective layer, which is not illustrated, may be disposed on the color filter. The color filter may also be disposed on the protective layer 6. Alternatively, the color filter may be disposed on an opposite substrate, such as a glass substrate, and then bonded.

A display apparatus 100 illustrated in Fig. 1B includes organic light-emitting devices 26 and TFTs 18 as an example of transistors. A substrate 11 made of, for example, glass or silicon, is provided, and an insulating layer 12 is disposed thereon. Active elements, such as TFTs 18, are arranged on the insulating layer. A gate electrode 13, a gate insulating film 14, and a semiconductor layer 15 of each of the active elements are arranged. Each active element 18 also includes a semiconductor layer 15, a drain electrode 16, and a source electrode 17. The active elements 18 are overlaid with an insulating film 19. An anode 21 included in the organic light-emitting devices 26 is coupled to the source electrodes 17 through contact holes 20 provided in the insulating film.

The mode of electrical connection between the electrodes (anode and cathode) included in each organic light-emitting device 26 and the electrodes (source electrode and drain electrode) included in a corresponding one of the TFTs is not limited to the mode illustrated in Fig. 1B. That is, it is sufficient that any one of the anode and the cathode is electrically coupled to any one of the source electrode and the drain electrode of the TFT. The TFTs refers to thin-film transistors.

In the display apparatus 100 illustrated in Fig. 1B, each organic compound layer is illustrated as a single layer; however, the organic compound layer 22 may be formed of multiple layers. A first protective layer 24 and a second protective layer 25 are disposed on the cathodes 23 in order to reduce the deterioration of the organic light-emitting devices.

Although the transistors are used as switching elements in the display apparatus 100 illustrated in Fig. 1B, other switching elements may be used instead.

The transistors used in the display apparatus 100 illustrated in Fig. 1B are not limited to transistors using a single-crystal silicon wafer, but may also be thin-film transistors including active layers on the insulating surface of a substrate. Examples of the active layer include single-crystal silicon, non-single-crystal silicon materials, such as amorphous silicon and microcrystalline silicon, and non-single-crystal oxide semiconductors, such as indium-zinc oxide and indium-gallium-zinc oxide. Thin-film transistors are also referred to as TFT elements.

The transistors in the display apparatus 100 illustrated in Fig. 1B may be formed in the substrate, such as a Si substrate. The expression "formed in the substrate" indicates that the transistors are produced by processing the substrate, such as a Si substrate. When the transistors are formed in the substrate, the substrate and the transistors can be deemed to be integrally formed.

In the organic light-emitting device according to the present embodiment, the luminance of emission light is controlled by the TFT elements, which are an example of switching elements; thus, an image can be displayed at respective luminance levels by arranging multiple organic light-emitting devices in the plane. The switching elements according to the present embodiment are not limited to the TFTs and may be low-temperature polysilicon transistors or active-matrix drivers formed on a substrate such as a Si substrate. The expression "on a substrate" can also be said to be "in the substrate". Whether transistors are formed in the substrate or TFTs are used is selected in accordance with the size of a display unit. For example, when the display unit has a size of about 0.5 inches, organic light-emitting devices are preferably disposed on a Si substrate.

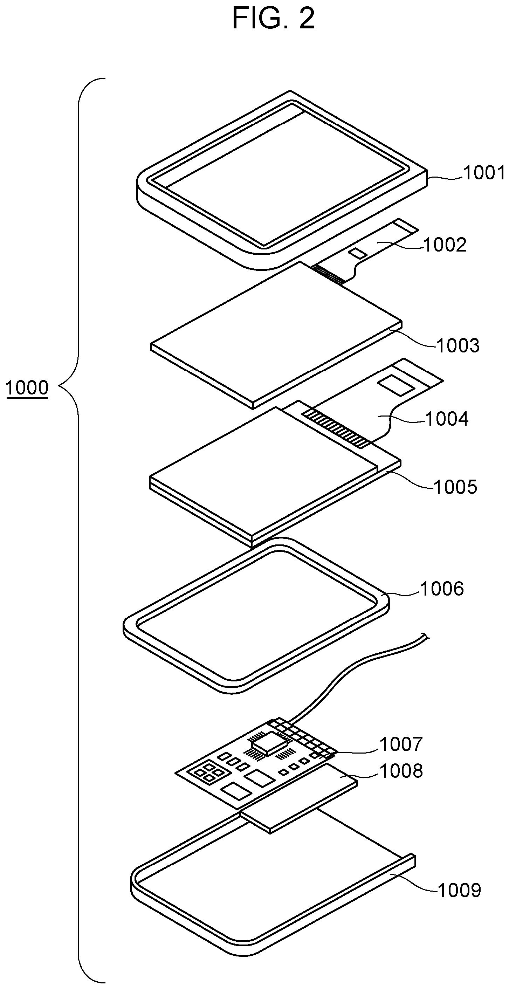

Fig. 2 is a schematic view illustrating an example of a display apparatus according to the present embodiment. A display apparatus 1000 may include, between an upper cover 1001 and a lower cover 1009, a touch panel 1003, a display panel 1005, a frame 1006, a circuit substrate 1007, and a battery 1008. The display panel 1005 may include the organic light-emitting device according to the present embodiment. The touch panel 1003 and the display panel 1005 are coupled to flexible printed circuits FPCs 1002 and 1004, respectively. The circuit substrate 1007 includes printed transistors. The battery 1008 need not be provided unless the display apparatus is a portable apparatus. The battery 1008 may be disposed at a different position even if the display apparatus is a portable apparatus.

The display apparatus according to the present embodiment may include a color filter having red, green, and blue portions. In the color filter, the red, green, and blue portions may be arranged in a delta arrangement.

The display apparatus according to the present embodiment may be used for the display unit of a portable terminal. In that case, the display apparatus may have both a display function and an operation function. Examples of the portable terminal include mobile phones, such as smartphones, tablets, and head-mounted displays.

The display apparatus according to the present embodiment may be used as a display unit of an image pickup apparatus including an image pickup device that receives light. The image pickup apparatus may include a display unit that displays information acquired by the image pickup device. The display unit may be a display unit exposed to the outside of the image pickup apparatus or may be a display unit disposed in a finder. The image pickup apparatus may be a digital camera or a digital camcorder.

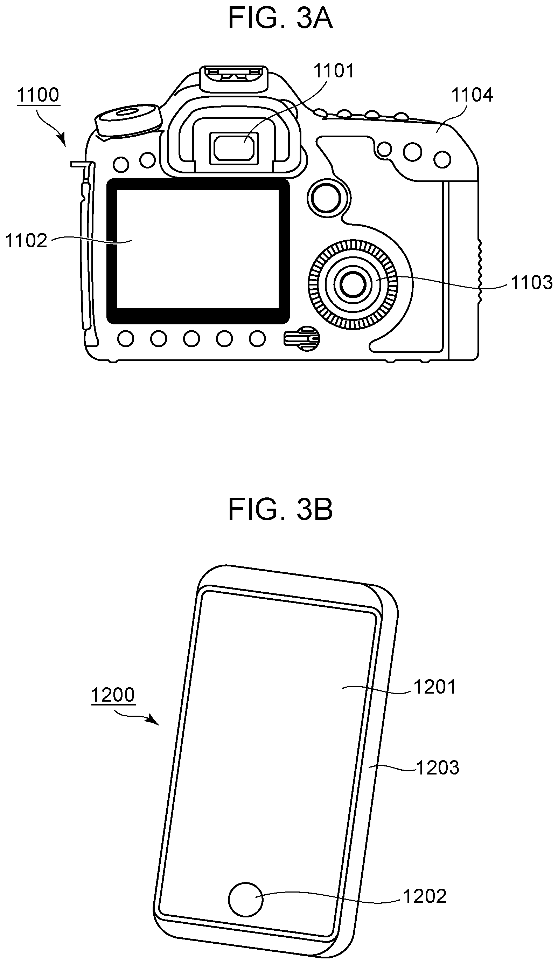

Fig. 3A is a schematic view illustrating an example of an image pickup apparatus according to the present embodiment. An image pickup apparatus 1100 may include a viewfinder 1101, a rear display 1102, an operation unit 1103, and a housing 1104. The viewfinder 1101 and the rear display 1102 may include the organic light-emitting device according to the present embodiment. In this case, the viewfinder 1101 and the rear display 1102 may display environmental information, imaging instructions, and so forth in addition to an image to be captured. The environmental information may include, for example, the intensity of external light, the direction of the external light, the moving speed of a subject, and the possibility that the subject will be shielded by an obstacle.

The timing suitable for imaging is only for a short time; thus, it is better to display the information as soon as possible. Accordingly, the display apparatus including the organic light-emitting device according to the present embodiment is preferably used. This is because organic light-emitting devices have a fast response time.

The image pickup apparatus 1100 may further include an optical unit, which is not illustrated. The optical unit may include a single lens or multiple lenses and is configured to form an image on an image pickup device in the housing 1104. The relative positions of the multiple lenses can be adjusted to adjust the focal point. This operation can also be performed automatically. The image pickup apparatus may also be referred to as a photoelectric conversion apparatus. Examples of an image capturing method employed in the photoelectric conversion apparatus can include a method for detecting a difference from the previous image and a method for cutting out an image from images always recorded, instead of sequentially capturing images.

Fig. 3B is a schematic view illustrating an example of an electronic apparatus according to the present embodiment. An electronic apparatus 1200 includes a display unit 1201, an operation unit 1202, and a housing 1203. The housing 1203 may accommodate a circuit, a printed circuit substrate including the circuit, a battery, and a communication unit. The operation unit 1202 may be a button or a touch-panel-type reactive unit. The operation unit may be a biometric recognition unit that recognizes fingerprints to perform functions, such as unlocking. An electronic apparatus including a communication unit can also be referred to as a communication apparatus. The electronic apparatus may further have a camera function by including a lens and an image pickup device. An image captured by the camera function is displayed on the display unit. Examples of the electronic apparatus include smartphones and laptop computers.

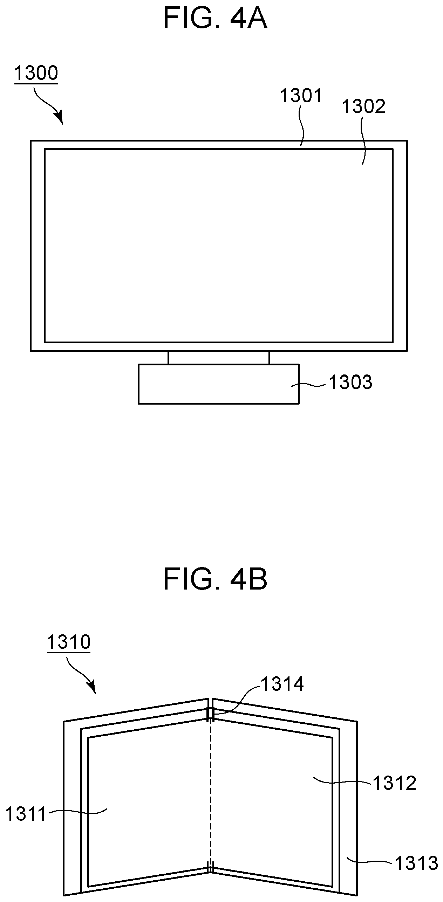

Figs. 4A and 4B are each a schematic view illustrating an example of a display apparatus according to the present embodiment. Fig. 4A illustrates a display apparatus, such as a television monitor or a PC monitor. A display apparatus 1300 includes a housing 1301 and a display unit 1302. The display unit 1302 may include the organic light-emitting device according to the present embodiment.

The display apparatus 1300 may include a base 1303 that supports the housing 1301 and the display unit 1302. The base 1303 is not limited to a form illustrated in Fig. 4A. The lower side of the housing 1301 may also serve as a base.

The housing 1301 and the display unit 1302 may be curved. The radius of curvature may be 5,000 mm or more and 6,000 mm or less.

Fig. 4B is a schematic view illustrating an example of a display apparatus according to the present embodiment. A display apparatus 1310 illustrated in Fig. 4B can be folded and is what is called a foldable display apparatus. The display apparatus 1310 includes a first display unit 1311, a second display unit 1312, a housing 1313, and an inflection point 1314. The first display unit 1311 and the second display unit 1312 may include the organic light-emitting device according to the present embodiment. The first display unit 1311 and the second display unit 1312 may be a single, seamless display apparatus. The first display unit 1311 and the second display unit 1312 can be divided from each other at the inflection point. The first display unit 1311 and the second display unit 1312 may display different images from each other. Alternatively, a single image may be displayed in the first and second display units.

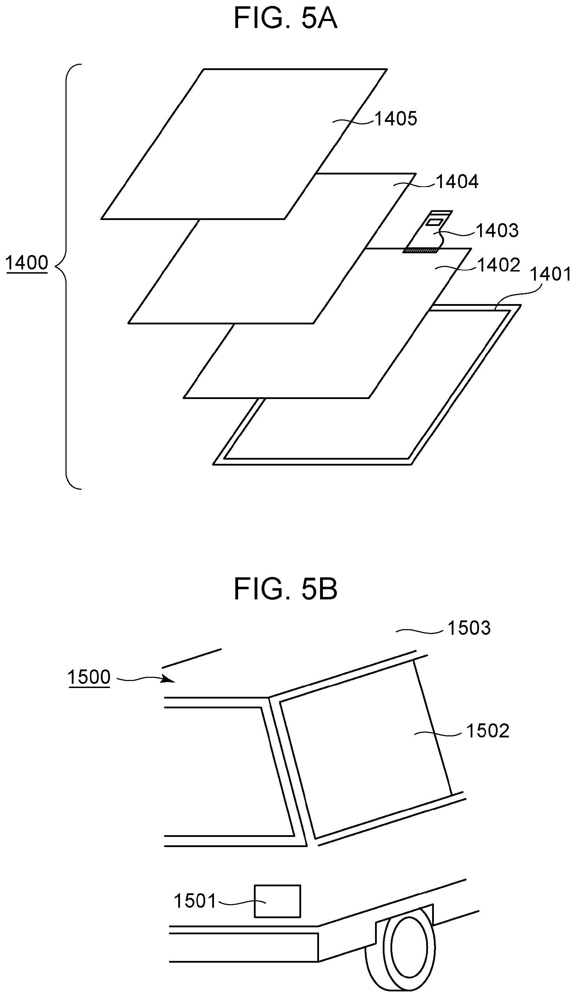

Fig. 5A is a schematic view illustrating an example of a lighting apparatus according to the present embodiment. A lighting apparatus 1400 may include a housing 1401, a light source 1402, and a circuit substrate 1403. The light source 1402 may include an organic light-emitting device according to the present embodiment. The lighting apparatus 1400 may include an optical film 1404 to improve the color rendering properties of the light source. The lighting apparatus 1400 may also include a light diffusion unit 1405 to effectively diffuse the light from the light source. When the lighting apparatus 1400 includes the light diffusion unit 1405, the apparatus can deliver light over a wide range. The optical film 1404 and the light diffusion unit 1405 may be provided on the light emission side of the lighting apparatus. A cover may be disposed at the outermost portion, as needed.

The lighting apparatus is, for example, an apparatus that lights a room. The lighting apparatus may emit light of white, neutral white, or any color from blue to red. The lighting apparatus according to the present embodiment may include a light control circuit that controls them. The lighting apparatus according to the present embodiment may also include a power supply circuit connected to the organic light-emitting device according to the present embodiment. The power supply circuit may be a circuit that converts an AC voltage into a DC voltage. The color temperature of white is 4200 K, and the color temperature of neutral white is 5000 K. The lighting apparatus according to the present embodiment may further include a color filter.

The lighting apparatus according to the present embodiment may include a heat dissipation unit. The heat dissipation unit is configured to release the heat inside the apparatus to the outside, and materials with high thermal conductivity, such as metals and ceramic materials, are used.

Fig. 5B is a schematic view of an automobile as an example of a moving object according to the present embodiment. The automobile includes a tail lamp, which is an example of lighting units. An automobile 1500 includes a tail lamp 1501 and an automobile body 1503, and may be configured to light the tail lamp when an operation, such as braking, is performed. The automobile body 1503 may also be referred to as a body. The automobile 1500 may include windows 1502 attached to the automobile body 1503. The tail lamp 1501 may include an organic light-emitting device according to the present embodiment. The tail lamp may include a protective member that protects the light source. The protective member may be made of any material as long as it has a certain degree of strength and is transparent. The protective member is preferably made of, for example, polycarbonate. The polycarbonate may be mixed with, for example, a furandicarboxylic acid derivative or an acrylonitrile derivative.

The windows 1502 may be transparent displays unless they are windows used to check areas in front of and behind the automobile. The transparent displays may include the organic light-emitting devices according to the present embodiment. In this case, the constituent materials, such as the electrodes, of the organic light-emitting devices according to the present invention are formed of transparent members.

The moving object according to the present embodiment includes one or both of a driving force generation unit configured to generate a driving force mainly used for movement of the moving object and a rotating body mainly used for movement of the moving object. The driving force generation unit may be an engine, a motor, or the like. The rotating body may be a tire, a wheel, a screw of a vessel, a propeller of an aircraft, or the like. Specifically, the moving object may be a bicycle, an automobile, a train, a vessel, an aircraft, a drone, or the like. The moving object may include a body and a lighting unit attached to the body. The lighting unit may emit light so that the position of the body can be recognized.

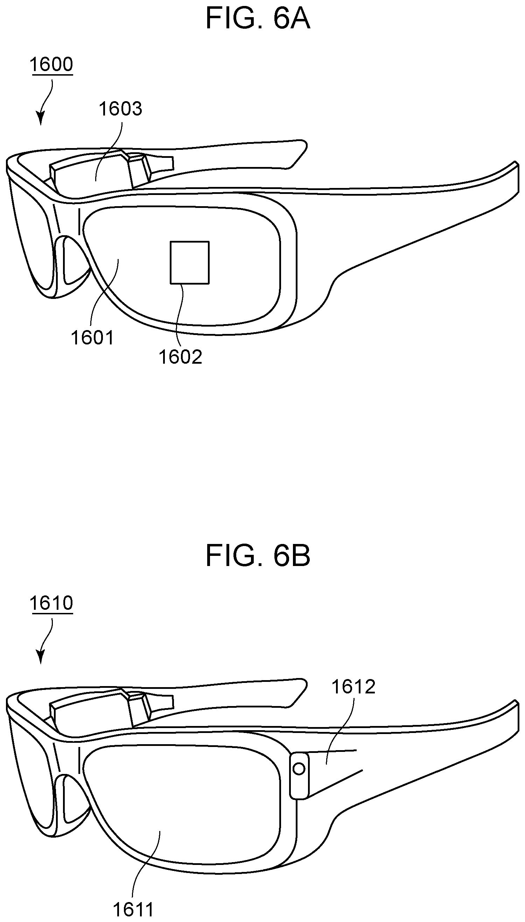

Examples of applications of the display apparatus of the above embodiment will be described with reference to Figs. 6A and 6B. The display apparatus can be used for systems that can be worn as wearable devices, such as smart glasses, head-mounted displays, and smart contact lenses. The display apparatus usable in a wearable device may include an image pickup apparatus that can photoelectrically convert visible light and a display apparatus that can emit visible light.

Figs. 6A and 6B are each a schematic view illustrating an example of glasses (smart glasses) according to the present embodiment. The glasses 1600 (smart glasses) will be described with reference to Fig. 6A. The glasses 1600 include a display unit on the rear surface side of a lens 1601. The display unit may include an organic light-emitting device according to the present invention. In addition, an image pickup apparatus 1602, such as a CMOS sensor or a SPAD, may be provided on the front surface side of the lens 1601.

The glasses 1600 further include a control unit 1603. The control unit 1603 functions as a power supply that supplies electric power to the image pickup apparatus 1602 and the display unit. The control unit 1603 controls the operation of the image pickup apparatus 1602 and the display unit. The lens 1601 includes an optical system for focusing light from the image pickup apparatus 1602 and the display unit.

Glasses 1610 (smart glasses) will be described with reference to Fig. 6B. The glasses 1610 include a control unit 1612. The control unit 1612 is provided with a display apparatus including an organic light-emitting device according to the present invention. The control unit 1612 may further include an image pickup apparatus equivalent to the image pickup apparatus 1602. The lens 1611 is provided with an optical system for projecting light emitted from the control unit 1612, and an image is projected onto the lens 1611. The control unit 1612 functions as a power supply that supplies electric power to the image pickup apparatus and the display apparatus and controls the operations of the image pickup apparatus and the display apparatus. The control unit may include a gaze detection unit that detects the gaze of a wearer. Infrared radiation may be used for gaze detection. An infrared light-emitting unit emits infrared light toward the eyeball of a user gazing at a displayed image. An image of the eyeball is captured by an image pickup unit including a light-receiving element detecting infrared light reflected from the eyeball among the emitted infrared light. A deterioration in image quality is reduced by providing a reduction unit that reduces light from the infrared light-emitting unit to the display unit when viewed in plan.

The control unit 1612 detects the user's gaze at the displayed image from the image of the eyeball captured with the infrared light. Any known method can be used for the gaze detection using the captured image of the eyeball. As an example, a gaze detection method based on a Purkinje image of the reflection of irradiation light on a cornea can be used.

More specifically, the gaze detection process is performed on the basis of a pupil-corneal reflection method. Using the pupil-corneal reflection method, the user's gaze is detected by generating a gaze vector representing the direction (rotation angle) of the eyeball based on the image of the pupil and the Purkinje image contained in the captured image of the eyeball.

A display apparatus according to the present embodiment may include an image pickup apparatus including light-receiving elements, and may control an image displayed on the display apparatus based on the gaze information of the user from the image pickup apparatus.

Specifically, in the display apparatus, a first field-of-view area at which the user gazes and a second field-of-view area other than the first field-of-view area are determined on the basis of the gaze information. The first field-of-view area and the second field-of-view area may be determined by the control unit of the display apparatus or may be determined by receiving those determined by an external control unit. In the display area of the display apparatus, the display resolution of the first field-of-view area may be controlled to be higher than the display resolution of the second field-of-view area. That is, the resolution of the second field-of-view area may be lower than that of the first field-of-view area.

The display area includes the first field-of-view area and the second field-of-view area different from the first field-of-view area. Based on the gaze information, an area of higher priority is determined from the first field-of-view area and the second field-of-view area. The first field-of-view area and the second field-of-view area may be determined by the control unit of the display apparatus or may be determined by receiving those determined by an external control unit. The resolution of an area of higher priority may be controlled to be higher than the resolution of an area other than the area of higher priority. In other words, the resolution of an area of a relatively low priority may be low.

Artificial intelligence (AI) may be used to determine the first field-of-view area and the high-priority field-of-view area. The AI may be a model configured to estimate the angle of gaze from the image of the eyeball and the distance to a target object located in the gaze direction, using the image of the eyeball and the actual direction of gaze of the eyeball in the image as teaching data. AI may be provided in the display apparatus, in the image pickup apparatus, or in an external device. When the external device includes AI, smart glasses that further include an image pickup apparatus that captures an external image can preferably be used. The smart glasses can display the captured external information in real time.

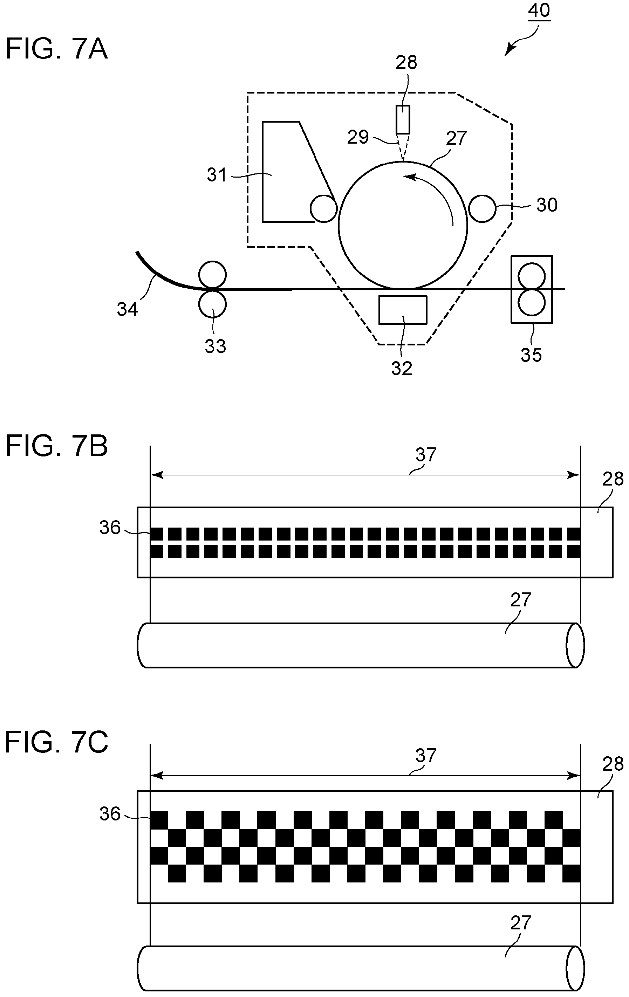

Fig. 7A is a schematic view of an example of an image-forming apparatus according to the present embodiment. An image-forming apparatus 40 is an electrophotographic image-forming apparatus and includes a photoconductor 27, an exposure light source 28, a charging unit 30, a developing unit 31, a transfer unit 32, a conveying roller 33, and a fixing unit 35. The irradiation of light 29 is performed from the exposure light source 28 to form an electrostatic latent image on the surface of the photoconductor 27. The exposure light source 28 may include the organic light-emitting device according to the present embodiment. The developing unit 31 contains, for example, a toner. The charging unit 30 charges the photoconductor 27. The transfer unit 32 transfers the developed image to a recording medium 34. The conveying roller 33 conveys the recording medium 34. The recording medium 34 is paper, for example. The fixing unit 35 fixes the image formed on the recording medium 34.

Figs. 7B and 7C each illustrate the exposure light source 28 and are each a schematic view illustrating multiple light-emitting portions 36 arranged on a long substrate. Arrows 37 each represent the row direction in which the organic light-emitting devices are arranged. The row direction is the same as the direction of the axis about which the photoconductor 27 rotates. This direction can also be referred to as the long-axis direction of the photoconductor 27. Fig. 7B illustrates a configuration in which the light-emitting portions 36 are arranged in the long-axis direction of the photoconductor 27. Fig. 7C is different from Fig. 7B in that the light-emitting portions 36 are arranged alternately in the row direction in a first row and a second row. The first row and the second row are located at different positions in the column direction. In the first row, the multiple light-emitting portions 36 are spaced apart. The second row has the light-emitting portions 36 at positions corresponding to the positions between the light-emitting portions 36 in the first row. In other words, the multiple light-emitting portions 36 are also spaced apart in the column direction. The arrangement in Fig. 7C can also be referred to as, for example, a gridlike arrangement, a staggered arrangement, or a checkered pattern.

As described above, the use of an apparatus including the organic light-emitting device according to the present embodiment enables a stable display with good image quality even for a long time.

### EXAMPLES

The present invention will be described below by examples. However, the present invention is not limited thereto.

### [Example 1 (Synthesis of Compound A-1)]

Compound A-1 was synthesized according to the following synthetic route.

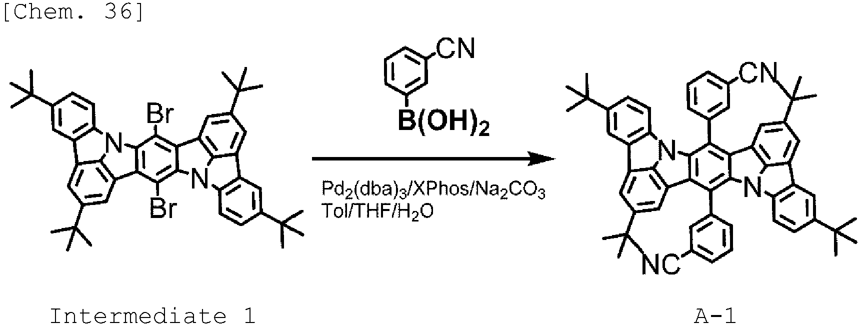

Intermediate 1 (0.300 g), (3-cyanophenyl)boronic acid (0.225 g), Pd₂(dba)₃ (0.014 g), XPhos (0.015 g), sodium carbonate (0.240 g), toluene (45 mL), tetrahydrofuran (45 mL), and water (15 mL) were added, and the mixture was stirred at 80°C for 16 hours. The reaction mixture was extracted with toluene, and the crude product was purified with an organic-solvent GPC column (JAIGEL-2H-40, Nippon Analytical Industry Co., Ltd.; eluent: chloroform) to give Compound A-1 (238 mg) as a yellow powder. THF was added to this yellow powder, and the resulting solution was subjected to mass spectrometry by ultra-high-performance liquid chromatography (UPLC)-MS, thereby leading to the identification of the compound (m/z = 830.435, C₆₀H₅₄N₄).

### [Example 2 (Synthesis of Compound A-2)]

Compound A-2 was synthesized according to the following synthetic route.

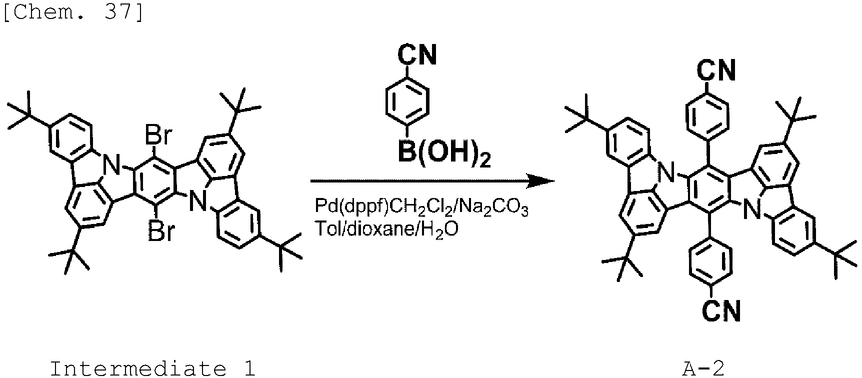

Intermediate 1 (0.400 g), (4-cyanophenyl)boronic acid (0.600 g), Pd(dppf)CH₂Cl₂ (0.033 g), sodium carbonate (0.323 g), toluene (100 mL), dioxane (100 mL), and water (30 mL) were added, and the mixture was stirred at 150°C for 16 hours. The reaction mixture was extracted with toluene, and the crude product was purified with an organic-solvent GPC column (JAIGEL-2H-40, Nippon Analytical Industry Co., Ltd.; eluent: chloroform) to give Compound A-2 (288 mg) as a yellow powder. THF was added to this yellow powder, and the resulting solution was subjected to mass spectrometry by ultra-high-performance liquid chromatography (UPLC)-MS, leading to the identification of the compound (m/z = 830.435, C₆₀H₅₄N₄).

### [Example 3 (Production and Evaluation of Organic Light-Emitting Device)]

In Example 3, Compound A-1 was used as a guest material in a light-emitting layer. An organic light-emitting device including an anode, a hole injection layer, a hole transport layer, an electron-blocking layer, a light-emitting layer, a hole-blocking layer, an electron transport layer, and a cathode provided in this order over a substrate was produced by a method described below.

An ITO film, serving as an anode, having a thickness of 100 nm was formed on a glass substrate by a sputtering method, and the resulting substrate was used as a transparent conductive supporting substrate (ITO substrate). The glass substrate with the ITO film was subjected to ultrasonic cleaning successively with acetone and isopropyl alcohol (IPA), washed with boiling IPA, and dried. Subsequently, the glass substrate was subjected to UV-ozone cleaning. The glass substrate treated in this manner was used as a transparent conductive supporting substrate.

Next, a hole injection layer, a hole transport layer, an electron-blocking layer, a light-emitting layer, a hole-blocking layer, an electron transport layer, and a metal electrode layer were successively deposited by vacuum evaporation using resistance heating in a vacuum chamber at 10⁻⁵ Pa. At this time, the formation was performed in such a manner that the electrode area was 3 mm². The layer structure is described below.

### [Table 6]

**Table 6**

| | Material | | | Thickness (nm) |
|---|---|---|---|---|
| Cathode | Metal electrode layer 2 | Al | | 100 |
| | Metal electrode layer 1 | Liq | | 0.5 |
| ETL | ET2 | | | 10 |
| HBL | ET12 | | | 20 |
| EML | Host | EM4 | Ratio by weight A-1:EM4 = 1:99 | 25 |
| | Guest | A-1 | | |
| EBL | HT31 | | | 10 |
| HTL | HT2 | | | 15 |
| HIL | HT16 | | | 5 |

In order not to cause the deterioration of the organic light-emitting device due to moisture adsorption, the resulting structure was covered with a protective glass plate in a dry air atmosphere and sealed with an acrylic resin adhesive. As described above, an organic light-emitting device was produced.

The characteristics of the resulting organic light-emitting device were measured and evaluated. The external quantum yield (EQE) of the organic light-emitting device at a current density of 10 mA/cm² was 4.46%.

Furthermore, a continuous operation test was performed at a current density of 20 mA/cm² to measure the time (LT80) when the percentage of luminance degradation reached 20% from the initial luminance. When the time at which the percentage of luminance degradation in Comparative Example 1 reached 20% is defined as 1.0, the relative value of LT80 of the present example was 2.41.

The full width at half maximum of the emission spectrum was determined to be 29 nm.

In the present example, the measuring instruments were as follows: specifically, the current-voltage characteristics were measured using a DC voltage-current source/monitor 6253 manufactured by ADC Corporation, and the luminance was measured using a spectroradiometer SR-LEDW from Topcon Corporation.

### [Example 4 and Comparative Example 1 (Production and Evaluation of Organic Light-Emitting Device)]

An organic light-emitting device of Example 4 was produced in the same manner as in Example 3, except that Compound A-1 was replaced with Compound A-2. An organic light-emitting device of Comparative Example 1 was produced in the same manner as in Example 3, except that Compound A-1 was replaced with Compound Ref-1. The characteristics of the organic light-emitting devices thus produced were measured and evaluated in the same manner as in Example 3. The results are presented in Table 7.

### [Table 7]

**Table 7**

| | Compound | EQE/% | LT80 (relative value) | Full width at half maximum /nm |
|---|---|---|---|---|
| Example 3 | A-1 | 4.46 | 2.41 | 29 |
| Example 4 | A-2 | 4.48 | 2.50 | 37 |
| Comparative Example 1 | Ref-1 | 4.45 | 1.00 | 49 |

As presented in Table 7, the organic compounds according to the present invention exhibited a superior device lifetime compared to Comparative Compound Ref-1. This is considered to be because each of the organic compounds according to the present invention has electron-withdrawing groups and thus has a lower HOMO level (i.e., further from the vacuum level). In addition, each of the organic compounds according to the present invention exhibited a smaller full width at half maximum than Comparative Compound Ref-1.

From the above, the organic compound according to the present invention is an organic compound having excellent oxidation stability. Therefore, when the organic compound according to the present invention is used in an organic light-emitting device, the organic light-emitting device having an excellent device lifetime can be provided. Furthermore, when an organic compound having excellent oscillator strength among the organic compounds according to the present invention is used in an organic light-emitting device, an organic light-emitting device having an excellent quantum yield (luminous efficiency) can be provided. In addition, when an organic compound having excellent color purity among the organic compounds according to the present invention is used in an organic light-emitting device, an organic semiconductor that emits light having even more excellent color purity can be provided.

The present invention can also have the following configurations.

### (Configuration 1)

An organic compound represented by Formula (1).

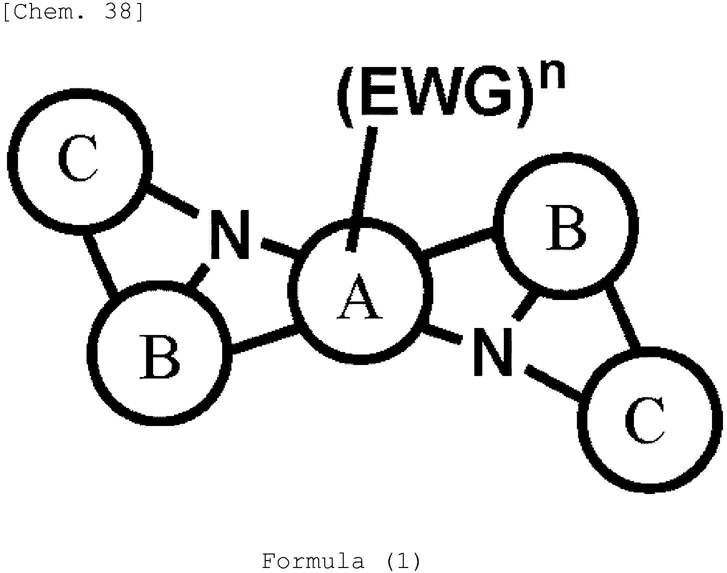

In Formula (1), Rings A to C are each independently selected from the group consisting of a substituted or unsubstituted aryl group having 6 or more and 50 or less carbon atoms and a substituted or unsubstituted heterocyclic group having 3 or more and 50 or less carbon atoms. When any of Rings A to C has a substituent, the substituent is a deuterium atom, an alkyl group, an aryl group, a heterocyclic group, a silyl group, an amino group, or a cyano group.

EWG is an electron-withdrawing group, an aryl group having at least one electron-withdrawing group, or a heterocyclic group having at least one or more electron-withdrawing groups.

n is an integer of 1 or more.

### (Configuration 2)

The organic compound described in configuration 1, in which, in Formula (1), the electron-withdrawing group is a trifluoromethyl group, a trichloromethyl group, a nitro group, a cyano group, an aldehyde group, a ketone group, an ester group, a carboxylic acid group, a sulfonyl group, a sulfonic acid group, a pyridyl group, a triazinyl group, or a halogen atom.

### (Configuration 3)

The organic compound described in configuration 1 or 2, in which, in Formula (1), the electron-withdrawing group is a trifluoromethyl group, a cyano group, or a fluorine atom.

### (Configuration 4)

The organic compound described in any one of configurations 1 to 3, in which, in Formula (1), EWG is an aryl group having at least one electron-withdrawing group and having 6 or more and 20 or less carbon atoms, or a heterocyclic group having at least one or more electron-withdrawing groups and having 3 or more and 17 or less carbon atoms.

### (Configuration 5)

The organic compound described in any one of configurations 1 to 4, in which, in Formula (1), the aryl group contained in EWG is benzene.

### (Configuration 6)

The organic compound described in any one of configurations 1 to 5, in which, in Formula (1), Rings A to C are each a substituted or unsubstituted aryl group having 6 or more and 20 or less carbon atoms and a substituted or unsubstituted heterocyclic group having 3 or more and 17 or less carbon atoms.

### (Configuration 7)

The organic compound described in any one of configurations 1 to 6, in which, in Formula (1), Ring A is a benzene skeleton or a naphthalene skeleton, and n is an integer of 1 or more and 4 or less.

### (Configuration 8)

The organic compound described in any one of configurations 1 to 7, in which, in Formula (1), each Ring B is a benzene skeleton, a benzofuran skeleton, or a benzothiophene skeleton.

### (Configuration 9)

The organic compound described in any one of configurations 1 to 8, in which, in Formula (1), each Ring C is a benzene skeleton, a benzofuran skeleton, or a benzothiophene skeleton.

### (Configuration 10)

An organic compound represented by Formula (2) or (3).

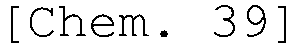

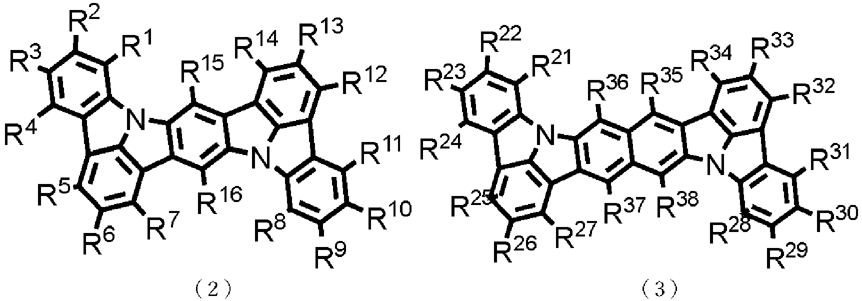

In Formula (2), R¹ to R¹⁶ are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted alkoxy group, a substituted or unsubstituted aryloxy group, a substituted or unsubstituted heteroaryloxy group, a substituted or unsubstituted silyl group, a substituted or unsubstituted amino group, and a cyano group. R¹ and R², R² and R³, R³ and R⁴, R⁵ and R⁶, R⁶ and R⁷, R⁸ and R⁹, R⁹ and R¹⁰, R¹⁰ and R¹¹, R¹² and R¹³, or R¹³ and R¹⁴ may be taken together to form a ring, provided that at least one of R¹⁵ and R¹⁶ is an electron-withdrawing group, an aryl group having at least one or more electron-withdrawing groups, or a heterocyclic group having at least one or more electron-withdrawing groups.

In Formula (3), R²¹ to R³⁸ are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted alkoxy group, a substituted or unsubstituted aryloxy group, a substituted or unsubstituted heteroaryloxy group, a substituted or unsubstituted silyl group, a substituted or unsubstituted amino group, and a cyano group. R²¹ and R²², R²² and R²³, R²³ and R²⁴, R²⁴ and R²⁵, R²⁶ and R²⁷, R²⁸ and R²⁹, R²⁹ and R³⁰, R³⁰ and R³¹, R³² and R³³, or R³³ and R³⁴ may be taken together to form a ring, provided that at least one of R³⁵ to R³⁸ is an electron-withdrawing group, an aryl group having at least one or more electron-withdrawing groups, or a heterocyclic group having at least one or more electron-withdrawing groups.

### (Configuration 11)

The organic compound described in configuration 10, in which, in each of Formulae (2) and (3), the electron-withdrawing group is a trifluoromethyl group, a trichloromethyl group, a nitro group, a cyano group, an aldehyde group, a ketone group, an ester group, a carboxylic acid group, a sulfonyl group, a sulfonic acid group, a pyridyl group, a triazinyl group, or a halogen atom.

### (Configuration 12)

The organic compound described in configuration 10 or 11, in which, in each of Formulae (2) and (3), the electron-withdrawing group is a trifluoromethyl group, a cyano group, or a fluorine atom.

### (Configuration 13)

The organic compound described in any one of configurations 10 to 12, in which, in each of Formulae (2) and (3), each ring is a substituted or unsubstituted aryl group having 6 or more and 12 or less carbon atoms or a substituted or unsubstituted heterocyclic group having 3 or more and 12 or less carbon atoms.

### (Configuration 14)

The organic compound described in any one of configurations 10 to 13, in which, in each of Formulae (2) and (3), each ring is a substituted or unsubstituted benzofuran skeleton or a substituted or unsubstituted benzothiophene skeleton.

### (Configuration 15)

An organic light-emitting device including a first electrode, a second electrode, and an organic compound layer disposed between the first electrode and the second electrode, in which the organic compound layer contains the organic compound described in any one of configurations 1 to 14.

### (Configuration 16)

The organic light-emitting device described in configuration 15, in which the organic compound layer includes a light-emitting layer, and the light-emitting layer contains the organic compound.

### (Configuration 17)

The organic light-emitting device described in configuration 16, in which the light-emitting layer further contains a first compound, and the lowest excited singlet energy of the first compound is higher than the lowest excited singlet energy of the organic compound.

### (Configuration 18)

The organic light-emitting device described in configuration 17, in which the first compound is a hydrocarbon compound containing a fused polycyclic hydrocarbon group.

### (Configuration 19)

The organic light-emitting device described in configuration 17 or 18, in which the light-emitting layer further contains a second compound, the lowest excited singlet energy of the second compound is higher than the lowest excited singlet energy of the organic compound and is lower than the lowest excited singlet energy of the first compound.

### (Configuration 20)

A display apparatus, including multiple pixels, in which at least one of the multiple pixels includes the organic light-emitting device described in any one of configurations 15 to 19 and a transistor coupled to the organic light-emitting device.

### (Configuration 21)

A photoelectric conversion apparatus, including an image pickup device configured to receive light, and a display unit configured to display an image captured by the image pickup device, in which the display unit includes the organic light-emitting device described in any one of configurations 15 to 19.

### (Configuration 22)

An image display apparatus, including a display unit including the organic light-emitting device described in any one of configurations 15 to 19, and a housing provided with the display unit.

### (Configuration 23)

An electronic apparatus, including a display unit including the organic light-emitting device described in any one of configurations 15 to 19, a housing provided with the display unit, and a communication unit disposed in the housing and configured to communicate with the outside.

### (Configuration 24)

A wearable device, including a display unit including the organic light-emitting device described in any one of configurations 15 to 19, an optical system configured to focus light from the display unit, and a control unit configured to control display of the display unit.

### (Configuration 25)

A lighting apparatus, including a light source including the organic light-emitting device described in any one of configurations 15 to 19, and a housing provided with the light source.

### (Configuration 26)

A moving object, including a lighting unit including the organic light-emitting device described in any one of configurations 15 to 19, and a body provided with the lighting unit.

### (Configuration 27)

An image-forming apparatus, including a photoconductor, and an exposure light source configured to expose the photoconductor, in which the exposure light source includes the organic light-emitting device described in any one of configurations 15 to 19.

The present invention is not limited to the above embodiments and various changes and modifications can be made within the spirit and scope of the present invention. Therefore, to apprise the public of the scope of the present invention, the following claims are made.

This application claims priority based on Japanese Patent Application No. 2023-145293 filed September 7, 2023, which is hereby incorporated by reference herein in its entirety.

### Reference Signs List

- 1: interlayer insulating layer
- 2: reflective electrode
- 3: insulating layer
- 4: organic compound layer
- 5: transparent electrode
- 6: protective layer
- 7: color filter
- 10: subpixel
- 11: substrate
- 12: insulating layer
- 13: gate electrode
- 14: gate insulating film
- 15: semiconductor layer
- 16: drain electrode
- 17: source electrode
- 18: thin-film transistor
- 19: insulating film
- 20: contact hole
- 21: lower electrode
- 22: organic compound layer
- 23: upper electrode
- 24: first protective layer
- 25: second protective layer
- 26: organic light-emitting device
- 27: photoconductor
- 28: exposure light source
- 29: light
- 30: charging unit
- 31: developing unit
- 32: transfer unit
- 33: conveying unit
- 34: recording medium
- 35: fixing unit
- 36: light-emitting portion
- 37: first direction parallel to long axis of photoconductor
- 40: image-forming apparatus
- 100: display apparatus
- 1000: display apparatus
- 1001: upper cover
- 1002: flexible printed circuit
- 1003: touch panel
- 1004: flexible printed circuit
- 1005: display panel
- 1006: frame
- 1007: circuit substrate
- 1008: battery
- 1009: lower cover
- 1100: image pickup apparatus
- 1101: viewfinder
- 1102: rear display
- 1103: operation unit
- 1104: housing
- 1200: electronic apparatus
- 1201: display unit
- 1202: operation unit
- 1203: housing
- 1300: display apparatus
- 1301: frame
- 1302: display unit
- 1303: base
- 1310: display apparatus
- 1311: first display unit
- 1312: second display unit
- 1313: housing
- 1314: inflection point
- 1400: lighting apparatus
- 1401: housing
- 1402: light source
- 1403: circuit substrate
- 1404: optical film
- 1405: light diffusion unit
- 1500: automobile
- 1501: tail lamp
- 1502: window
- 1503: automobile body
- 1600: smart glasses
- 1601: lens
- 1602: image pickup apparatus
- 1603: control unit
- 1610: smart glasses
- 1611: lens
- 1612: control unit

## Claims

1. An organic compound represented by Formula (1): 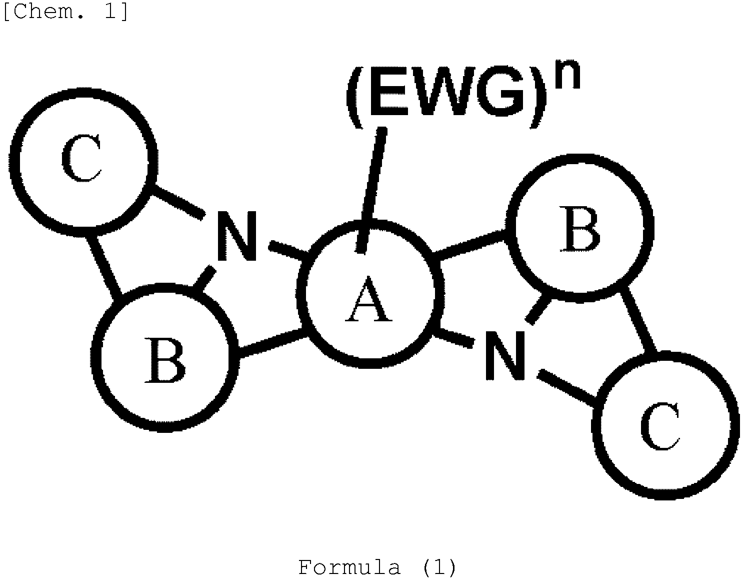 wherein, in Formula (1), Rings A to C are each independently selected from the group consisting of a substituted or unsubstituted aryl group having 6 or more and 50 or less carbon atoms and a substituted or unsubstituted heterocyclic group having 3 or more and 50 or less carbon atoms, when any of Rings A to C has a substituent, the substituent is a deuterium atom, a halogen atom, an alkyl group, an aryl group, a heterocyclic group, an alkoxy group, an aryloxy group, a heteroaryloxy group, a silyl group, an amino group, or a cyano group,
EWG is an electron-withdrawing group, an aryl group having at least one electron-withdrawing group, or a heterocyclic group having at least one or more electron-withdrawing groups, and
n is an integer of 1 or more.

2. The organic compound according to claim 1, wherein, in Formula (1), the electron-withdrawing group is a trifluoromethyl group, a trichloromethyl group, a nitro group, a cyano group, an aldehyde group, a ketone group, an ester group, a carboxylic acid group, a sulfonyl group, a sulfonic acid group, a pyridyl group, a triazinyl group, or a halogen atom.

3. The organic compound according to claim 1, wherein, in Formula (1), the electron-withdrawing group is a trifluoromethyl group, a cyano group, or a fluorine atom.

4. The organic compound according to claim 1, wherein, in Formula (1), EWG is an aryl group having at least one electron-withdrawing group and having 6 or more and 20 or less carbon atoms, or a heterocyclic group having at least one or more electron-withdrawing groups and having 3 or more and 17 or less carbon atoms.

5. The organic compound according to claim 1, wherein, in Formula (1), the aryl group contained in EWG is benzene.

6. The organic compound according to claim 1, wherein, in Formula (1), Rings A to C are each a substituted or unsubstituted aryl group having 6 or more and 20 or less carbon atoms and a substituted or unsubstituted heterocyclic group having 3 or more and 17 or less carbon atoms.

7. The organic compound according to claim 1, wherein, in Formula (1), Ring A is a benzene skeleton or a naphthalene skeleton, and n is an integer of 1 or more and 4 or less.

8. The organic compound according to claim 1, wherein, in Formula (1), each Ring B is a benzene skeleton, a benzofuran skeleton, or a benzothiophene skeleton.

9. The organic compound according to claim 1, wherein, in Formula (1), each Ring C is a benzene skeleton, a benzofuran skeleton, or a benzothiophene skeleton.

10. An organic compound represented by Formula (2) or (3): 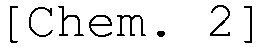 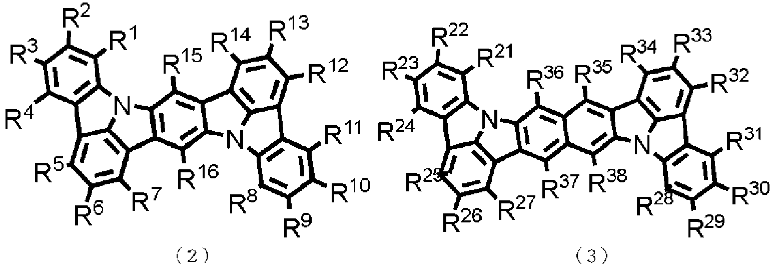 wherein, in Formula (2), R¹ to R¹⁶ are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted alkoxy group, a substituted or unsubstituted aryloxy group, a substituted or unsubstituted heteroaryloxy group, a substituted or unsubstituted silyl group, a substituted or unsubstituted amino group, and a cyano group, and R¹ and R², R² and R³, R³ and R⁴, R⁵ and R⁶, R⁶ and R⁷, R⁸ and R⁹, R⁹ and R¹⁰, R¹⁰ and R¹¹, R¹² and R¹³, or R¹³ and R¹⁴ are optionally taken together to form a ring, provided that at least one of R¹⁵ and R¹⁶ is an electron-withdrawing group, an aryl group having at least one or more electron-withdrawing groups, or a heterocyclic group having at least one or more electron-withdrawing groups, and
wherein, in Formula (3), R²¹ to R³⁸ are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted alkoxy group, a substituted or unsubstituted aryloxy group, a substituted or unsubstituted heteroaryloxy group, a substituted or unsubstituted silyl group, a substituted or unsubstituted amino group, and a cyano group, and R²¹ and R²², R²² and R²³, R²³ and R²⁴, R²⁴ and R²⁵, R²⁶ and R²⁷, R²⁸ and R²⁹, R²⁹ and R³⁰, R³⁰ and R³¹, R³² and R³³, or R³³ and R³⁴ are optionally taken together to form a ring, provided that at least one of R³⁵ to R³⁸ is an electron-withdrawing group, an aryl group having at least one or more electron-withdrawing groups, or a heterocyclic group having at least one or more electron-withdrawing groups.

11. An organic light-emitting device, comprising a first electrode, a second electrode, and an organic compound layer disposed between the first electrode and the second electrode, wherein the organic compound layer contains the organic compound according to Claim 1.

12. The organic light-emitting device according to claim 11, wherein the organic compound layer includes a light-emitting layer, and the light-emitting layer contains the organic compound.

13. The organic light-emitting device according to claim 12, wherein the light-emitting layer further contains a first compound, and a lowest excited singlet energy of the first compound is higher than a lowest excited singlet energy of the organic compound.

14. The organic light-emitting device according to claim 13, wherein the first compound is a hydrocarbon compound containing a fused polycyclic hydrocarbon group.

15. The organic light-emitting device according to claim 13, wherein the light-emitting layer further contains a second compound, a lowest excited singlet energy of the second compound is higher than the lowest excited singlet energy of the organic compound and is lower than the lowest excited singlet energy of the first compound.

16. A display apparatus, comprising multiple pixels, at least one of the multiple pixels including the organic light-emitting device according to any one of claims 11 to 15 and a transistor coupled to the organic light-emitting device.

17. A photoelectric conversion apparatus, comprising an image pickup device configured to receive light, and a display unit configured to display an image captured by the image pickup device, wherein the display unit includes the organic light-emitting device according to any one of claims 11 to 15.

18. An image display apparatus, comprising a display unit including the organic light-emitting device according to any one of claims 11 to 15, and a housing provided with the display unit.

19. An electronic apparatus, comprising a display unit including the organic light-emitting device according to any one of claims 11 to 15, a housing provided with the display unit, and a communication unit disposed in the housing and configured to communicate with the outside.

20. A wearable device, comprising a display unit including the organic light-emitting device according to any one of claims 11 to 15, an optical system configured to focus light from the display unit, and a control unit configured to control display of the display unit.

21. A lighting apparatus, comprising a light source including the organic light-emitting device according to any one of claims 11 to 15, and a housing provided with the light source.

22. A moving object, comprising a lighting unit including the organic light-emitting device according to any one of claims 11 to 15, and a body provided with the lighting unit.

23. An image-forming apparatus, comprising a photoconductor, and an exposure light source configured to expose the photoconductor, wherein the exposure light source includes the organic light-emitting device according to any one of claims 11 to 15.
